(19) Europäisches Patentamt
European Patent Office
Office européen des brevets

(11) EP 3 233 190 B1

(12) **FASCICULE DE BREVET EUROPEEN**

(45) Date de publication et mention de la délivrance du brevet:
**24.02.2021 Bulletin 2021/08**

(21) Numéro de dépôt: **15820290.3**

(22) Date de dépôt: **17.12.2015**

(51) Int Cl.:
*A61K 31/341* (2006.01) *A61K 31/7004* (2006.01)
*A61K 31/7028* (2006.01) *A61P 31/04* (2006.01)
*A01N 43/08* (2006.01) *A23L 29/00* (2016.01)
*A61Q 17/00* (2006.01) *A61L 2/00* (2006.01)
*C11D 3/43* (2006.01) *C07C 41/09* (2006.01)
*C07H 15/04* (2006.01) *C07D 307/20* (2006.01)
*A23L 3/34* (2006.01) *A61K 8/33* (2006.01)
*C07D 407/04* (2006.01) *C07D 493/04* (2006.01)
*A23L 5/00* (2016.01) *A61P 11/02* (2006.01)
*A61P 17/00* (2006.01) *A61P 31/02* (2006.01)
*A23L 3/3463* (2006.01)

(86) Numéro de dépôt international:
**PCT/IB2015/059733**

(87) Numéro de publication internationale:
**WO 2016/098048 (23.06.2016 Gazette 2016/25)**

(54) **COMPOSITION ANTIBACTÉRIENNE COMPRENANT UN ACÉTAL OU UN ÉTHER D'HEXITANE À LONGUE CHAÎNE ALKYLE**

ANTIBAKTERIELLE ZUSAMMENSETZUNG MIT EINEM ACETAL ODER LANGKETTIGEN ALKYLHEXITANETHER

ANTIBACTERIAL COMPOSITION COMPRISING AN ACETAL OR A LONG-CHAIN ALKYL HEXITANE ETHER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2014 FR 1402894**

(43) Date de publication de la demande:
**25.10.2017 Bulletin 2017/43**

(73) Titulaires:
- **TEREOS STARCH & SWEETENERS BELGIUM**
  **9300 Aalst (BE)**
- **Université Claude Bernard Lyon 1**
  **69622 Villeurbanne Cedex (FR)**
- **Centre National de la Recherche Scientifique**
  **75016 Paris (FR)**

(72) Inventeurs:
- **GOZLAN, Charlotte**
  **93190 Livry Gargan (FR)**
- **BELMESSIERI, Dorine**
  **74500 Evian Les Bains (FR)**
- **DUCLOS, Marie-Christine**
  **69622 Villeurbanne CEDEX (FR)**
- **DUGUET, Nicolas**
  **69622 Villeurbanne CEDEX (FR)**
- **LEMAIRE, Marc**
  **69622 Villeurbanne CEDEX (FR)**
- **LINA, Gérard**
  **69622 Villeurbanne Cedex (FR)**
- **DUMITRESCU, Oana**
  **69004 Lyon (FR)**
- **REDL, Andreas**
  **9300 Aalst (BE)**

(74) Mandataire: **Icosa**
**83 avenue Denfert-Rochereau**
**75014 Paris (FR)**

(56) Documents cités:
**EP-A1- 0 092 998**
**WO-A1-2012/148530**
**WO-A1-2014/025413**
**WO-A1-2014/199345**
**WO-A1-2015/189796**
**WO-A1-2016/088076**
**JP-A- H1 115 114**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- PETER KÖLL ET AL: "Carbohydrate-Based Liquid Crystals: Mesogenic 1-O-Alkyl Derivatives of 2,5-Anhydrohexitols", ANGEWANDTE CHEMIE. INTERNATIONAL EDITION., vol. 25, no. 4, 1 avril 1986 (1986-04-01), pages 368-369, XP055249595, DE ISSN: 0570-0833, DOI: 10.1002/anie.198603681
- SHUICHI MATSUMURA ET AL: "Surface activities, biodegradability and antimicrobial properties of n-alkyl glucosides, mannosides and galactosides", JOURNAL OF THE AMERICAN OIL CHEMISTS' SOCIETY, vol. 67, no. 12, 1 décembre 1990 (1990-12-01), pages 996-1001, XP055029089, ISSN: 0003-021X, DOI: 10.1007/BF02541865
- Anonymous: "Sorbitan PubChem", , 25 February 2020 (2020-02-25), XP055737950, Retrieved from the Internet: URL:https://pubchem.ncbi.nlm.nih.gov/compound/Sorbitan [retrieved on 2020-10-08]

## Description

### Domaine technique

La présente invention concerne une composition bactéricide ou bactériostatique comprenant un acétal d'alkyle ou un éther d'alkyle de sorbitane à longue chaîne alkyle pour son utilisation dans le traitement ou la prévention des infections à bactéries Gram positif, son utilisation en tant que produit d'hygiène ou dermatologique à usage externe ainsi qu'une méthode de désinfection de surfaces.

### Arrière-plan technique

Les composés antimicrobiens se définissent comme des molécules capables d'inhiber ou de stopper la croissance de micro-organismes ou de les tuer. Dans ce contexte, ils sont couramment utilisés pour prévenir ou traiter les infections humaines et animales, et dans l'industrie agroalimentaire pour prévenir la multiplication de bactéries pathogènes dans les aliments. La large gamme d'utilisation des composés antimicrobiens a favorisé l'émergence d'agents infectieux résistants. La diffusion de bactéries ayant acquis des mécanismes de résistance vis-à-vis des composés antimicrobiens les plus largement utilisés sont un problème de santé publique majeur de plus en plus alarmant (J. S. Bradley et al. Lancet Infect. Dis. 2007;7:68-78).

À titre illustratif, de nombreuses souches résistantes aux antibiotiques de l'espèce la plus pathogène du genre Staphylococcus, à savoir *Staphylococcus aureus* ont été isolées. Or, les infections à staphylocoques représentent un pourcentage important des infections graves. De plus, près de la moitié des infections nosocomiales seraient liées à un staphylocoque. On peut citer également les nombreuses souches d'*Enterococcus faecalis* ou *d'Enterococcus faecium* résistantes aux agents antibiotiques communément utilisés. Or, bien qu'elles ne soient moins virulentes par rapport aux staphylocoques notamment, on recense un nombre croissant de souches d'entérocoques multirésistantes et plus récemment des épidémies d'entérocoques résistant aux glycopeptides, les antibiotiques de recourt vis-à-vis de cette famille bactérienne.

Un autre phénomène d'antibiorésistance a été décrit qui pourrait ne pas être seulement lié à l'usage excessif d'antibiotiques, mais aux méthodes de conservation des aliments. Ainsi, par exemple il a été montré que *Listeria monocytogenes* s'avère plus résistante aux antibiotiques après avoir survécu à un stress osmotique, à une basse température ou à un milieu acide (Anas A. et al. (2015) Food Microbiology, Volume 46, April, Pages 154-160). Or, la contamination humaine est d'origine alimentaire. En outre, bien qu'elle soit relativement rare, la listériose humaine est une infection grave avec une mortalité estimée à 50 %. Ainsi, l'émergence de résistance aux antibiotiques chez L. *monocytogenes* qui pourrait être induite par les méthodes modernes de conservation ou de traitement des aliments constitue une grave menace pour la santé publique.

Bien que plusieurs mécanismes soient souvent impliqués simultanément dans la résistance aux antibiotiques, il est commun de les classer en trois catégories: (a) défaut de pénétration de l'antibiotique dans la bactérie, (b) inactivation ou excrétion de l'antibiotique par des systèmes enzymatiques bactériens et (c) défaut d'affinité entre cible bactérienne et antibiotique. Ces trois catégories de mécanisme de résistance ont une composante structurelle à savoir les mécanismes mis en œuvre sont dépendants de la structure de la molécule concernée.

Ainsi, afin d'obtenir une composition antibiotique ayant des chances réduites de permettre le développement d'une résistance, les inventeurs ont envisagé l'utilisation d'une composition contenant un mélange de composés ayant une activité antibiotique mais comportant des différences structurelles mineures susceptibles de réduire les chances de développement d'une résistance bactérienne. Ils ont ainsi envisagé une composition comprenant un mélange isomérique de composés ayant une activité antibiotique.

Les inventeurs ont souhaité développer une composition antibiotique ayant également une faible toxicité et un faible impact environnemental. Une composition antibiotique biodégradable susceptible d'être obtenue en grandes quantités à partir de ressources renouvelables, à faible coût afin d'être parfaitement accessibles pour une application industrielle mais également aussi efficaces que des antimicrobiens non biosourcés.

Or, aucun procédé de l'art antérieur ne permet d'obtenir un mélange isomérique de composés biosourcés à faible toxicité et à faible coût.

Néanmoins, des composés biosourcés ont été décrits par l'art antérieur. Ainsi, l'art antérieur décrit différents composé utilisés comme antimicrobiens parmi lesquels les acides gras et leurs correspondants d'esters polyhydroxylés actifs contre les bactéries Gram positif et possédant de longues chaînes aliphatiques. A titre indicatif, un des antimicrobiens les plus actifs est la monolaurine, un monoester de glycérol présentant une chaîne aliphatique en C12. Sa dénomination commerciale est la LAURICIDIN®. Ce composé est utilisé en tant qu'additif alimentaire dans le but d'inhiber la croissance bactérienne (E. Freese, C. W. Sheu, E. Galliers. Nature 1973, 241, 321-325; E. G. A. Verhaegh, D. L. Marshall, D.-H. Oh. Int. J. Food Microbiol. 1996, 29, 403-410). Or, la fonction ester de la monolaurine étant sensible aux estérases, ce composé est rapidement dégradé et possède un temps de demi vie faible.

L'art antérieur décrit également des antimicrobiens dérivés de sucre considérés comme particulièrement attractifs du fait de leur biodégradabilité, leurs faibles toxicités et impact environnemental.

Des exemples d'antimicrobiens dérivés de sucre sont les esters dérivés de sucre qui sont également utilisés industriellement pour des applications antimicrobiennes car leurs matières premières et leur coût de production restent relativement peu élevés. On peut citer par exemple, le caprylate de sorbitane décrit dans la demande internationale de brevet WO2014/025413 en mélange avec de l'Hinokitiol dans une formulation antimicrobienne. Selon cette demande, cette formulation permettrait d'inhiber ou de tuer des bactéries à Gram positif et négatif, des champignons et/ou des mycoses.

L'art antérieur décrit également l'utilisation d'esters de disaccharide en tant qu'agent antimicrobien dans l'industrie alimentaire. Le dodécanoyl de sucrose est l'un des plus utilisés. Ce dernier serait particulièrement actif contre la *L. monocytogenes* (M. Ferrer, J. Soliveri, F.J. Plou, N. López-Cortés, D. Reyes-Duarte, M. Christensen, J.L. Copa-Patiño, A. Ballesteros, Enz. Microb. Tech., 2005, 36, 391-398). Néanmoins, il est également décrit comme faiblement inhibiteur de la croissance de *S. aureus,* pour des applications dans le domaine hospitalier (J.D. Monk, L.R. Beuchat, A.K. Hathcox, J. Appl. Microbiol., 1996, 81, 7-18). Ainsi, l'ester de sucrose présenterait des propriétés bactériostatiques (arrête la croissance bactérienne) mais pas bactéricides (tue les bactéries).

En outre, la synthèse d'esters de sucre présente de nombreux inconvénients. Tout d'abord, malgré le faible coût de production, la synthèse d'esters, plus particulièrement pour les di- et trisaccharides, est problématique à cause de la haute fonctionnalité des sucres qui entraîne la formation d'un mélange de mono-, di- et polyesters et la présence de solvant polaire, comme le diméthylformamide (DMF) et la pyridine, est généralement nécessaire afin de mieux solubiliser les réactifs hautement polaires. Cependant, ces solvants sont classés Cancérigène, Mutagène et Reprotoxique (CMR) et leur usage doit être évité. Pour résoudre ce problème, la synthèse enzymatique a été utilisée mais la nécessité de se placer en milieu très dilué dans ces conditions, rend la production limitée.

Par ailleurs, les fonctions esters de ces composés sont facilement hydrolysables par les estérases présentes dans les cellules. Or, les molécules libérées suite à cette hydrolyse à savoir, le sucre et l'acide gras, ne présentent pas ou peu de propriétés antimicrobiennes (l'acide gras étant légèrement actif). Ceci induit une instabilité responsable d'une réduction du temps d'activité de ces composés.

Ainsi, afin d'obtenir une composition antibiotique peu propice au développement d'une résistance comprenant des agents antimicrobiens efficaces et stables, l'invention propose un acétal d'alkyl ou un éther d'alkyl de sorbitane à longue chaîne alkyle ayant un très bonne activité antimicrobienne que ce soit sous sa forme pure ou en mélange d'isomères, un tel produit pouvant être obtenu notamment dans des conditions peu onéreuses tout en étant respectueuses pour l'environnement et ne représentant pas de danger pour des applications topiques ou par ingestion.

**Description détaillée de l'invention**

Composition bactéricide ou bactériostatique

L'invention concerne une composition bactéricide ou bactériostatique pour son utilisation dans le traitement ou la prévention des infections bactériennes par des bactéries à Gram positif. La composition comprend un mélange de régioisomères et/ ou de diastéréoisomères d'un acétal d'alkyle ou un éther d'alkyle de sorbitane, dans lequel le groupe alkyle comprend entre 11 à 18 atomes de carbone, un sel pharmaceutiquement acceptable. Ledit radical acétal alkyle ou éther alkyle est en position 2-0, 3-0, 5-0 et/ou 6-0. Le radical acétal alkyle est en position 2,3-*O*; 3,5-*O* ou 5,6-*O*.

Le terme "sels pharmaceutiquement acceptables" désigne n'importe quel sel qui, par administration au patient est capable de fournir (directement ou indirectement) un composé comme celui décrit présentement. La préparation de sels peut être effectuée par des procédés connus dans l'état de la technique.

Le terme « hexitane » désigne le produit obtenu par la déshydratation d'un hexose hydrogéné (ou hexitol) tel qu'un sorbitol ou un mannitol. Typiquement, l'hexitane désigne le sorbitane, l'arlitane ou le mannitane. L'hexitane peut désigner le 1,4-anhydro-D-sorbitol (1,4-arlitane ou sorbitane); le 1,5-anhydro-D-sorbitol (polygalitol); le 3,6-anhydro-D-sorbitol (3,6-sorbitane); le 1,4 (3,6) -anhydro-D-mannitol (mannitan); le 1,5-anhydro-D-mannitol (styracitol); le 3,6-anhydro-D-galactitol; le 1,5-anhydro-D-galactitol; le 1,5-anhydro-D-talitol et le 2,5-anhydro-L-iditol.

Selon la présente invention, l'hexitane est le sorbitane.

Un tel acétal de sorbitane peut être obtenu par des méthodes connues de l'homme du métier telles que l'acétalisation directe, la trans-acétalisation par exemple. En outre, un éther d'alkyle de sorbitane peut être obtenu par des méthodes connues de l'homme du métier telles que la synthèse d'éther de Williamson, l'ouverture d'époxyde, la condensation d'alcools, la télomérisation d'alcools, la réduction d'acétals, l'alkylation réductrice directe ou indirecte.

Les inventeurs ont développé une méthode particulièrement avantageuse permettant l'obtention de ce dérivé sous la forme pure ou sous la forme d'un mélange d'isomères de monoéthers ou de monoacétals d'alkyle d'hexitane, préférentiellement les isomères sont des isomères de position et/ou de diastéréoisomères.

La composition bactéricide ou bactériostatique comprenant un monoéther ou un monoacétal d'alkyle de sorbitane ou un mélange d'isomères ce celui-ci préférentiellement de position peut être obtenue par un procédé comprenant les étapes suivantes :

a) une déshydratation d'un sorbitol pour obtenir un monoanhydrosorbitol;
b) une acétalisation ou trans-acétalisation du sorbitol ou du monoanhydrosorbitol obtenu en a) par un aldéhyde aliphatique contenant de 11 à 18 atomes de carbone ou l'acétal de celui-ci, typiquement par

   i. un aldéhyde aliphatique contenant de 11 à 18 atomes de carbone, par acétalisation, ou
   ii. un dérivé d'un d'aldéhyde aliphatique contenant de 11 à 18 atomes de carbone, par trans-acétalisation;

c) optionnellement, hydrogénolyse catalytique de l'acétal alkyle de sorbitane obtenue en b) préférentiellement, sans catalyseur acide
d) récupération d'un mélange d'isomères de monoéthers d'alkyle de sorbitane obtenue en c) dans lequel le groupe alkyle (R) comprend entre 11 et 18 atomes de carbone
ou
e) récupération d'un mélange d'isomères de monoacétals d'alkyle de sorbitane obtenue en b) dans lequel le groupe alkyle (R) comprend entre 11 à 18 atomes de carbone, et
f) optionnellement, purification de l'un ou l'autre des mélanges obtenus en d), notamment par chromatographie.

Typiquement, le dérivé d'un aldéhyde aliphatique peut être un acétal de di-alkyle de l'aldéhyde correspondant. Les acétals de di-méthyle et les acétals de di-éthyle sont préférés.

Un « monoanhydrohexitol » ou un « monoanhydrosorbitol » doit être compris comme étant obtenu par déshydratation, par l'élimination d'une ou plusieurs molécules d'eau à partir d'un hexitol, notamment du sorbitol. Un exemple monoanhydrosorbitol approprié peut être le 1,4-anhydro-D-sorbitol (1,4-arlitan ou sorbitan); 1,5-anhydro-D-sorbitol (polygalitol) ou le 3,6-anhydro-D-sorbitol (3,6-sorbitan).

Le monoanhydrosorbitol préféré est dérivé de la déshydratation du sorbitol pour former par exemple, le 1,4-sorbitane, le 3,6-sorbitane ou le 2,5-sorbitane.

Par « isomère de position » on entend des régioisomères, plus particulièrement on entend des isomères de monoéthers ou de monoacétals d'alkyle d'hexitane et notamment de sorbitane dans lesquels le radical monoéther ou monoacétal d'alkyl est positionné sur différent carbone de l'hexitane. Typiquement, des isomères de position du monoacétal d'alkyle de sorbitane sont le 2,3-*O*-; 3,5-*O*- ou 5,6-*O*- monoacétal alkyle de sorbitane.

Le terme «diastéréoisomères» désigne des isomères optiques qui ne sont pas superposables, ni image l'un de l'autre dans un miroir. Des exemples, de diastéréoisomères de monoacétal alkyle de sorbitane sont :

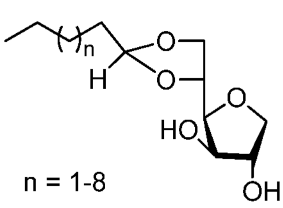
n = 1-8

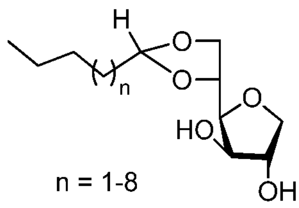
n = 1-8

Selon un mode de réalisation, le procédé selon l'invention peut comprendre une étape de déshydratation du sorbitol afin d'obtenir un monoanhydrosorbitol. Typiquement, le sorbitol est fondu avant l'étape de déshydratation. L'étape de déshydratation peut être effectuée avec un catalyseur par exemple, avec un catalyseur acide.

Selon l'invention, l'étape de déshydratation est effectuée sous atmosphère d'hydrogène à une pression de préférence d'environ de 20 à 50 bar.

Avantageusement, l'étape de déshydratation est effectuée à une température comprise entre 120 et 170°C, de préférence entre 130 et 140°C.

Typiquement, le sorbitol est purifié après l'étape de déshydratation, par exemple par cristallisation, recristallisation ou chromatographie.

L'étape d'acétalisation ou de trans-acétalisation comprend:

i) éventuellement, une étape de préchauffage du sorbitol, de préférence à une température comprise entre 70 et 130°C, typiquement entre 90 et 110°C,
ii) une étape d'addition de l'aldéhyde aliphatique ou d'un dérivé d'aldéhyde aliphatique audit sorbitol et
iii) une étape d'addition d'un catalyseur de préférence d'un catalyseur acide.

L'étape i) est particulièrement avantageuse en ce qu'elle peut être mise en œuvre en l'absence de solvant.

De préférence, le catalyseur acide utilisé durant l'étape d'acétalisation ou de trans-acétalisation et le cas échéant lors de l'étape de déshydratation peut être un catalyseur acide homogène ou hétérogène. Le terme «homogène», tel qu'utilisé dans l'expression « catalyseur acide homogène » se réfère à un catalyseur qui se trouve dans la même phase (solide, liquide ou gaz) ou dans le même état d'agrégat que le réactif. Inversement, le terme « hétérogène », tel qu'utilisé dans l'expression « catalyseur acide hétérogène » se réfère à un catalyseur qui se trouve dans une phase différente (solide, liquide ou gaz) que les réactifs.

Ledit catalyseur acide utilisé durant l'étape d'acétalisation ou de trans-acétalisation et le cas échéant lors de l'étape de déshydratation peut être indépendamment choisi parmi les acides solides ou liquides, organiques ou inorganiques, les acides solides étant préférés. En particulier, le catalyseur acide préféré est choisi parmi l'acide para-toluène sulfonique, l'acide méthane sulfonique, l'acide camphosulfonique (CSA) et les résines sulfoniques.

Typiquement, l'étape d'acétalisation ou de trans-acétalisation est effectuée à des températures comprises entre 70 et 130°C, typiquement entre 70 et 90°C. La température des mélanges réactionnels peut varier en fonction des réactifs et solvants utilisés. Le temps de réaction est déterminé par le degré de conversion atteint.

Selon un mode de réalisation, l'étape d'acétalisation ou de trans-acétalisation peut être effectuée par un aldéhyde aliphatique ou l'acétal de celui-ci, typiquement, un aldéhyde aliphatique linéaire ou ramifié ou l'acétal de celui-ci. L'étape d'acétalisation ou de trans-acétalisation peut être typiquement réalisée avec un aldéhyde aliphatique ou l'acétal de celui-ci ayant 11, 12, 13, 14, 15, 16, 17 ou 18 atomes de carbone, par exemple choisi parmi le undécanal, le dodécanal, le tridécanal, le tétradécanal, le pentadecanal, l'hexadécanal, l'heptadecanal, l'octodécanalet l'acétal. De préférence, l'aldéhyde aliphatique en C11-C13 ou l'acétal de celui-ci est un aldéhyde aliphatique en C12 ou l'acétal de celui-ci par exemple, un dodécanal ou l'acétal de celui-ci.

L'expression « l'acétal de celui-ci » ou « leur(s) acétal(s) », telle qu'utilisée présentement englobe le di-alkyl acétal de l'aldéhyde aliphatique en C11-C18 correspondant. Plus particulièrement, les acétals de di-méthyle ou de di-éthyle de l'aldéhyde aliphatique en C11-C18 sont préférés.

Selon un mode de réalisation, l'étape d'acétalisation ou de trans-acétalisation peut être effectuée avec ou sans solvant. Lorsque la réaction est effectuée en présence d'un solvant, le solvant est de préférence un solvant polaire.

Typiquement, le solvant peut être choisi parmi le diméthylformamide (DMF), le diméthylsulfoxyde (DMSO), le diméthylacétamide (DMA), l'acétonitrile ($CH_3CN$), le tétrahydrofurane (THF), le 2-méthyltétrahydrofuranne (2Me-THF), l'éther méthylique de cyclopentyle (CPME), le methanol (MeOH), l'éthanol (EtOH), le propanol (PrOH), l'isopropanol (iPrOH), le butanol (BuOH), l'éther dibutylique (DBE), le méthyle tert-butyle éther (MTBE) et le triméthoxypropane (TMP).

Des travaux expérimentaux approfondis ont conduit à une sélection de conditions permettant l'observation de taux de conversion et de rendements optimaux au cours des étapes d'acétalisation ou de trans-acétalisation. Les meilleurs résultats ont été obtenus lorsque le rapport molaire [(aldéhyde aliphatique en C11-C18 ou leur acétal) : monosaccharide] est compris entre 5:1 et 1:5, de préférence entre 4:1 et 1:4, et de manière avantageuse entre 3:1 et 1:3.

Les inventeurs ont montré plus particulièrement, que lors d'une réaction d'acétalisation, le rapport molaire aldéhyde aliphatique en C11-C18: monosaccharide compris entre 1:1 et 1:5 , de préférence entre 1:1 et 1:4, et de manière préférée entre 1:3 et 1:2 améliore les rendements et fournit des taux optimaux de conversion.

Les inventeurs ont montré en outre qu'au cours des réactions de trans-acétalisation, un rapport molaire acétal aliphatique en C11-C18 : monosaccharide compris entre 1:1 et 5:1, de préférence entre 5:4 et 4:1, de préférence entre 3:1 et 4:3, de préférence encore entre 3:2 et 2:5 améliore les rendements et fournit des taux optimaux de conversion. Les catalyseurs utilisés sont les mêmes que lors de la réaction d'acétalisation.

Un tel procédé de l'invention peut comprendre en outre au moins une étape de neutralisation et/ou de filtration et/ou de purification après l'une quelconque des étapes de déshydratation le cas échant, d'acétalisation ou de trans-acétalisation.

Lorsqu'une étape de purification est prévue, ladite étape de purification peut être par exemple, une cristallisation, une recristallisation ou une chromatographie. De préférence, la chromatographie est réalisée en utilisant un solvant polaire non-aqueux. En général, quand une étape de filtration et/ou de purification est prévue avant une étape optionnelle d'hydrogénolyse, le solvant polaire non-aqueux peut être identique à celui utilisé lors de l'étape d'hydrogénolyse.

Typiquement, la composition est bactéricide ou bactériostatique vis-à-vis des bactéries à Gram positif.

Avantageusement, la composition bactéricide ou bactériostatique est incorporée dans une composition alimentaire, cosmétique, pharmaceutique, phytosanitaire, vétérinaire ou de traitement de surface. Telle que par exemple, une composition cosmétique et/ou dermatologique de nettoyage et/ou de soin pour la peau, en particulier sous la forme d'une crème, un gel, une poudre, une lotion, un beurre notamment, un gel douche, savon, shampoing, bain de douche, déodorant, anti-transpirant, lingette humide, formulation d'écran solaire ou formulation cosmétique décorative.

L'invention concerne également, une **utilisation** d'une composition bactéricide ou bactériostatique selon l'invention pour son utilisation **comme produit d'hygiène ou dermatologique à usage externe.**

Typiquement un « **produit d'hygiène** » se réfère à tout produit utilisé pour le nettoyage, la désinfection ou l'hygiène, y compris par exemple une lotion, mousse, spray et liquide mais également des lingettes ou tout support

susceptible d'être imprégné de la composition selon l'invention. L'expression « **produit dermatologique** » se réfère à tout produit destiné à une application sur la peau ou les muqueuses.

Utilisation dans le traitement ou la prévention d'une infection à bactéries Gram positif.

L'invention concerne de plus une composition selon l'invention pour une **utilisation dans le traitement ou à la prévention des infections bactériennes par des bactéries à** Gram **positif.**

Par « traitement », on entend traitement à titre curatif (visant à au moins réduire, éradiquer ou stopper le développement de l'infection) chez un patient. Par « prévention », on entend traitement à titre prophylactique (visant à réduire le risque d'apparition de l'infection) chez un patient.

Le «patient» peut-être, par exemple un être humain ou un mammifère non-humain (par exemple un rongeur (souris, rat), un félin, un chien ou un primate) affecté par ou susceptible d'être affecté par des infections bactériennes et notamment à Gram positif. De préférence, le sujet est un humain.

L'expression « Gram-positif » se réfère à des bactéries qui sont colorées en bleu foncé ou le violet par la coloration de Gram, par opposition aux bactéries gram-négatives qui ne peuvent pas retenir le colorant violet. La technique de coloration Gram repose sur les caractéristiques membranaires et de paroi de la bactérie.

Typiquement, les bactéries à Gram positif sont des bactéries de **l'embranchement** des *Firmicutes,* typiquement de la **classe** des *Bacilli* notamment choisies parmi les bactéries de **l'ordre** des *Lactobacillales* ou des *Bacillales.*

Selon une forme de réalisation de l'invention, les bactéries de l'ordre des *Bacillales* sont choisies parmi la famille des *Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea* et *Turicibacteraceae*

Typiquement, les bactéries de la famille des *Listeriaceae* sont par exemple du genre des *Brochothrix* ou des *Listeria* et peuvent être typiquement, choisies parmi *L. fleischmannii, L. grayi, L. innocua, L. ivanovii, L. marthii, L. monocytogenes, L. rocourtiae, L. seeligeri, L. weihenstephanensis et L. welshimeri.*

Lorsque les bactéries à Gram positif sont des bactéries de la famille des *Staphylococcaceae,* elles sont notamment choisies parmi les bactéries du genre des *Staphylococcus, Gemella, Jeotgalicoccus, Macrococcus, Salinicoccus et Nosocomiicoccus.*

Les bactéries du genre des *Staphylococcus* par exemple choisies parmi S. *arlettae, S. agnetis, S. aureus, S. auricularis, S. capitis, S. caprae, S. carnosus, S. caseolyticus, S. chromogenes, S. cohnii, S. condimenti, S. delphini, S. devriesei, S. epidermidis. S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri et S. xylosus.*

Selon une autre forme de réalisation de l'invention, les bactéries de l'ordre des *Lactobacillales* sont choisies parmi une famille des *Aerococcaceae, Carnobacteriaceae, Enterococcaceae, Lactobacillaceae, Leuconostocaceae et Streptococcaceae.*

Typiquement, les bactéries de la famille des *Enterococcaceae* sont choisies parmi les bactéries du genre des *Bavariicoccus, Catellicoccus, Enterococcus, Melissococcus, Pilibacter, Tetragenococcus, Vagococcus.*

Les bactéries du genre des *Enterococcus* sont choisies par exemple, parmi *E. malodoratus, E. avium, E. durans, E. faecalis. E. faecium, E. gallinarum, E. hirae, E. solitarius, préférentiellement, E. avium, E. durans, E. faecalis et E. faecium.*

Les bactéries du genre des *Staphylococcus* et plus particulièrement *S. aureus* sont responsables de nombreuses infections de la peau ou des muqueuses telles que la muqueuse vaginale ou nasale. Par exemple, des infections telles que la folliculite, les abcès, les panaris, les furoncles, l'impétigo, les infections interdigitales, l'anthrax (anthrax staphylococcique), les cellulites, les surinfections de plaies, les otitis sinusitis, l'hydradénite, les mastites infectieuses, les infections post-traumatiques de la peau ou les infections de la peau brûlée.

Les bactéries du genre des *Enterococcus* et notamment *E. faecalis* sont responsables notamment d'endocardites, d'infections de la vessie, de la prostate ou de l'épididyme

L'**infection bactérienne par des bactéries à Gram positif** concerne préférentiellement une infection de la peau ou de muqueuses. L'utilisation telle que décrite ci-dessous concerne l'administration préférentiellement en topique, à un individu qui en a besoin, d'une quantité thérapeutiquement efficace de la composition selon l'invention.

Chez une personne infectée par une bactérie à Gram positif, on entend par « **quantité thérapeutiquement efficace** » une quantité suffisante pour empêcher l'infection d'évoluer vers une aggravation, voire suffisante pour faire régresser l'infection. Chez une personne non infectée, la « **quantité thérapeutiquement efficace** » est la quantité suffisante pour protéger une personne qui serait mise en contact avec une bactérie à Gram positif et éviter la survenue de l'infection causée par cette bactérie à Gram positif.

Typiquement, l'administration topique s'effectue par application sur la peau ou sur les muqueuses de la composition selon l'invention.

Méthode de désinfection ou de prévention de la colonisation bactérienne d'un substrat

L'invention porte en outre sur une méthode de désinfection ou de prévention de la colonisation bactérienne par des bactéries à Gram positif d'un substrat comprenant la mise en contact du substrat avec une composition selon l'invention.

Typiquement, un substrat est tout support susceptible d'être colonisé par des bactéries à Gram positif et susceptible de transmettre l'infection à un animal par contact ou par ingestion.

Par exemple, le substrat peut être un aliment d'origine végétale ou animale ou une composition alimentaire comprenant de tels aliments ou un extrait de ces aliments et notamment des céréales, des fruits, des légumes, de la viande, du poisson, des abats.

Le substrat peut être également un ou plusieurs éléments sélectionnés parmi des métaux, des plastiques, du verre, du béton, de la pierre.

Préférentiellement le substrat est un ustensile, un outil ou un appareil utilisé dans l'industrie alimentaire, (ustensiles de cuisine, contenant, système de conservation à froid, réfrigérateur, chambres froides..) en milieu hospitalier, tels que par exemple des outils de chirurgie ou des prothèses ou dans les transports en commun (barre de maintien transport en commun, sièges...).

Alternativement, l'invention concerne également l'utilisation d'une composition pour la désinfection, l'épuration, la stérilisation ou la purification des surfaces selon la méthode ci-dessous.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

L'invention sera mieux comprise à la lecture des figures et exemples suivants donnés uniquement à titre d'exemple.

**Exemples**

Les acétals de sorbitane ont été préparés par acétalisation ou transacétalisation des sucres suivant la procédure préalablement décrite dans le brevet N°13/01375 « Procédé pour la préparation d'acétals cycliques alkyl à longues chaînes, à base de sucres ». Les acétals de sucre sont ensuite réduits utilisant des conditions de réduction sans catalyseur acide préalablement décrite dans le brevet N°14/01346. A titre indicatif, la synthèse d'acétals et d'éthers de sorbitane est détaillée ci-dessous.

EXEMPLE 1 : **Procédure générale pour la synthèse d'acétals de sorbitane (A)**

*La déshydratation du sorbitol:*

Le D-sorbitol (20 g, 110 mmol) et 0,1% en moles d'acide camphorsulfonique sont ajoutés dans un autoclave de 150 mL en acier inoxydable. Le réacteur est hermétiquement fermé, purgé trois fois avec de l'hydrogène puis l'hydrogène a été introduit jusqu'à une pression de 50 bars. Le système est ensuite chauffé à 140 °C et agité avec un agitateur mécanique pendant 15 heures. Après refroidissement à température ambiante, la pression d'hydrogène a été libérée et la brut réactionnel a été diluée dans de l'éthanol (200 mL) pour obtenir un mélange homogène jaune. Le solvant est évaporé sous pression réduite et le résidu est ensuite cristallisé à partir de méthanol froid et filtré sous vide. Le matériau cristallin a été lavé avec du méthanol froid pour donner le 1,4-sorbitane (5,88 g, 35% sur théorique) sous forme d'un solide blanc. La pureté est de > 98%, telle que déterminée par HPLC, tandis que les cristaux ont montré un point de fusion de 113-114 °C. Le degré de conversion de la réaction a été déterminée à 73%, grâce à quoi on obtient un mélange de sorbitol, de 1,4-sorbitane, d'isosorbide et de quelques sous-produits en quantité très limitée, de sorte que le rapport du 1,4-sorbitane: isosorbide a été déterminé comme étant de 80: 20.

*Procédure générale pour l'acétalisation du sorbitane*

Dans un ballon à fond rond équipé d'un condenseur et d'une garde de $CaCl_2$, sous atmosphère d'argon, le 1,4-D-sorbitane (5.00 g, 30.5 mmol, 3 equiv) est dissout dans l'éthanol sec (15 mL). L'aldéhyde (10.2 mmol, 1 equiv) est ensuite ajouté goutte-à-goutte suivi de l'acide camphorsulfonique (CSA, 10 % massique par rapport à l'aldéhyde). Le mélange réactionnel est chauffé à 80°C pendant 15 heures sous agitation magnétique. Le mélange réactionnel est refroidi et le solvant est évaporé sous pression réduite. Le résidu est trituré dans l'acétate d'éthyle et l'excès de sorbitane est éliminé par filtration et lavé avec de l'acétate d'éthyle froid. Cette opération peut être répétée pour éliminer toute

traces de sorbitane. Le filtrat a été concentré sous pression réduite. Le résidu est purifié par chromatographie sur colonne de gel de silice pour donner les acétals alkyle de sorbitane. La composition du mélange de régioisomères 5,6-*O*-alkylidene- et 3,5-*O*-alkylidene-1,4-D-sorbitane a été déterminé par HPLC. De plus, chaque régioisomère a été obtenu sous la forme d'un mélange de deux diastéréoisomères.

*Procédure pour la trans-acetalisation du sorbitane :*

Dans un ballon à fond rond du 1,4-sorbitane (0,5 g, 3 mmol) a été dissout dans de l'éthanol (7,5 mL) et le 1,1-diethoxypentane (1,15 mL, 6 mmol) a été ajouté sous un flux d'argon, puis de l'acide camphosulfonique (50 mg; 10% p/p). Le mélange est chauffé à 80 °C et sous agitation magnétique. Après 3 heures, le mélange a été neutralisé et concentré sous pression réduite. Le résidu a été purifié par chromatographie flash (acétate d'éthyle / cyclohexane 80:20 à 100:0) pour donner l'acétal de sorbitane (0,43 g, 66% de rendement isolé) sous forme d'une huile incolore. L'HPLC a révélé un mélange de 4 isomères.

Exemple 1a :

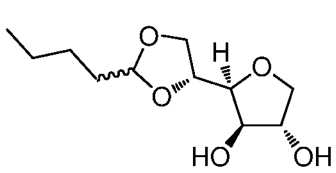
+
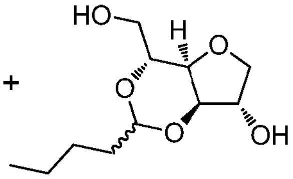

**1a + 1a'** **1a" + 1a'"**

**5,6-*O*-Pentylidene-1,4-D-sorbitan 1a** et **1a'** et **3,5-*O*-pentylidene-1,4-D-sorbitan 1a"** et **1a'":** Les composés ont été obtenus à partir du 1,4-D-sorbitane (0.49 g, 3 mmol) et du valéraldéhyde (0.107 mL, 1 mmol) selon la procédure générale **(A).** Après réaction, le résidu est purifié par chromatographie sur colonne de gel de silice (EtOAc/cyclohexane 80:20 → 100:0) pour donner un mélange 43:57 de régioisomères d'acétals de sorbitane en position 5,6-*O*- et 3,5-*O*- (0.189 g, 81%) sous la forme d'une huile incolore. Le produit obtenu est un mélange de régioisomères 5,6-*O*- et 3,5-*O*-d'acétals de sorbitane, chaque régioisomères étant un mélange de diastéréoisomères (26:17:47:10) comme déterminé par HPLC. RMN $^1H$ (300 MHz, $d_6$-DMSO) $\delta_H$ pour tous les isomères: 0.85 (3H, t, $J$ = 7.2), 1.16-1.35 (4H, m), 1.35-1.60 (2H, m), 3.30-4.30 (8H, sorbitan protons), 4.67-5.33 (3H, 3m, $^1H$ acetal et 2 OH); RMN $^{13}C$ (75 MHz, $d_6$-DMSO) $\delta_c$ pour les régioisomères 5,6-*O*-**1a** et **1a'** : 13.90 ($CH_3$), 22.06 ($CH_2$), 25.68 et 25.81 ($CH_2$), 33.16 ($CH_2$), 66.59 et 66.93 ($CH_2$), 72.79 et 73.19 (CH), 73.43 ($CH_2$), 75.46 et 75.68 (CH), 76.55 et 76.61 (CH), 80.74 et 81.01 (CH), 103.29 et 103.37 (CH); $\delta_c$ pour les régioisomères 3,5-*O*- **1a"** et **1a'":** 13.92 et 13.93 ($CH_3$), 21.95 et 22.00 ($CH_2$), 25.53 et 25.75 ($CH_2$), 33.73 et 34.13 ($CH_2$), 60.78 et 61.92 ($CH_2$), 72.37 et 73.55 ($CH_2$), 72.58 et 72.99 (CH), 73.19 et 73.96 (CH), 74.87 et 76.45 (CH), 78.38 et 79.08 (CH), 93.83 et 96.06 (CH); IR ν max: 3386 (OH), 2954, 2873, 1716, 1412, 1145, 1461, 1061, 1029, 967; HRMS (ESI+) calculé $C_{11}H_{20}NaO_5$: 255.1208 $[M+Na]^+$; mesuré : 255.1203 (+1.8 ppm); HPLC (isocratique 80:20 $H_2O/CH_3CN$ + 0.1% $H_3PO_4$): Rtpour les régioisomères 3,5-*O*- = 9.70 min **(1a",** 47%) et 11.25 min **(1a'",** 10%); $R_t$ pour les régioisomères 5,6-*O*- = 12.50 min **(1a,** 26%) et 14.49 **(1a',** 17%).

Exemple 1b :

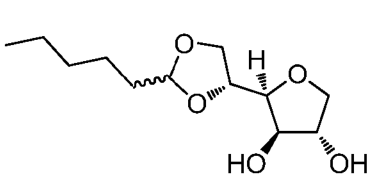
+
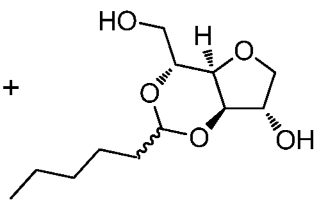

**1b + 1b'** **1b" + 1b'"**

**5,6-*O*-Hexylidene-1,4-D-sorbitan 1b** et **1b'** et **3,5-*O*-hexylidene-1,4-D-sorbitan 1b"** et **1b'":** Les composés

ont été obtenus à partir du 1,4-D-sorbitane (0.49 g, 3 mmol) et de l'hexanal (0.124 mL, 1 mmol) selon la procédure générale **(A).** Après réaction, le résidu est purifié par chromatographie sur colonne de gel de silice (EtOAc/cyclohexane 80:20 → 100:0) pour donner un mélange 57:43 de régioisomères d'acétals de sorbitane en position 5,6-*O*- et 3,5-*O*- (0.144 g, 58%) sous la forme d'une huile jaune. Le produit obtenu est un mélange de régioisomères 5,6-*O*- et 3,5-*O*- d'acétals de sorbitane, chaque régioisomères étant un mélange de diastéréoisomères (32:25:31:12) comme déterminé par HPLC. RMN $^1H$ (300 MHz, $d_6$-DMSO) $\delta_H$ pour tous les isomères: 0.85 (3H, t, $J$ = 6.5), 1.12-1.40 (6H, m), 1.45-1.58 (2H, m), 3.30-4.30 (8H, m, sorbitan protons), 4.72-4.90 (1H, m, acetal proton), 5.07-5.28 (2H, 2m, OH); RMN $^{13}C$ (75 MHz, $d_6$-DMSO) $\delta_c$ pour les régioisomères 5,6-*O*- **1b** and **1b':** 13.91 ($CH_3$), 22.12 ($CH_2$), 23.24 et 23.38 ($CH_2$), 31.24 ($CH_2$), 33.50 ($CH_2$), 66.64 et 66.98 ($CH_2$), 72.86 et 73.24 (CH), 73.48 ($CH_2$), 75.50 et 75.73 (CH), 76.60 et 76.66 (CH), 80.78 et 81.06 (CH), 103.34 et 103.42 (CH); $\delta_c$ pour les régioisomères 3,5-*O*- **1b"** and **1b'":** 13.93 ($CH_3$), 22.12 ($CH_2$), 23.09 et 23.31 ($CH_2$), 31.17 ($CH_2$), 34.06 et 34.48 ($CH_2$), 60.85 et 61.97 ($CH_2$), 72.42 et 73.61 ($CH_2$), 72.64 et 72.86 (CH), 73.08 et 74.01 (CH), 74.94 et 76.48 (CH), 78.40 et 79.13 (CH), 93.90 et 96.13 (CH); IR ν max: 3386 (OH), 2929 ($CH_3$), 2871 ($CH_2$), 2360, 2341, 1465, 1407, 1143, 1034; HRMS ($ESI^+$): $[M+Na]^+$ $C_{12}H_{22}NaO_5$ calculé 269.1359, mesuré 269.1360 (-0.4 ppm); HPLC (isocratique 80:20 $H_2O/CH_3CN$ + 0.1% $H_3PO_4$): $R_t$ pour les régioisomères 3,5-*O*- = 20.77 min (**1b",** 31%) et 24.65 min (**1b''',** 12%); $R_t$ pour les régioisomères 5,6-0- = 28.28 min (**1b,** 32%) et 33.90 (**1b',** 25%).

Exemple 1c :

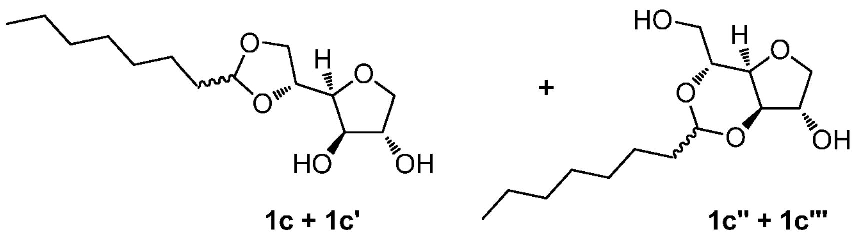

**1c + 1c'** **1c" + 1c'"**

**5,6-*O*-Octylidene-1,4-D-sorbitan 1c** et **1c'** et **3,5-*O*-octylidene-1,4-D-sorbitan 1c"** et **1c'":** Les composés ont été obtenus à partir du 1,4-D-sorbitane (1.00 g, 6 mmol) et de l'octanal (0.317 mL, 2 mmol) selon la procédure générale **(A).** Après réaction, le résidu est purifié par chromatographie sur colonne de gel de silice (EtOAc/cyclohexane 60:40 → 100:0) pour donner un mélange 61:39 de régioisomères d'acétals de sorbitane en position 5,6-*O*- et 3,5-*O*- (0.102 g, 37%)sous la forme d'une pâte blanche. Le produit obtenu est un mélange de régioisomères 5,6-*O*- et 3,5-*O*- d'acétals de sorbitane, chaque régioisomères étant un mélange de diastéréoisomères (32:29:28:11) comme déterminé par HPLC. RMN $^1H$ (300 MHz, $d_6$-DMSO) $\delta_H$ pour tous les isomères: 0.86 (3H, t, $J$ = 8.7), 1.10-1.42 (10H, m), 1.43-1.62 (2H, m), 3.38-4.31 (8H, m, sorbitan protons), 4.70-4.90 (1H, m, acetal proton), 5.02-5.28 (2H, 2m, OH); RMN $^{13}C$ (75 MHz, $d_6$-DMSO) $\delta_c$ pour les régioisomères 5,6-*O*- **1c** et **1c':** 13.96 ($CH_3$), 22.13 ($CH_2$), 23.40 et 23.58 ($CH_2$), 28.72 (2 $CH_2$), 31.26 ($CH_2$), 33.54 ($CH_2$), 66.22 et 66.96 ($CH_2$), 72.85 et 73.24 (CH), 73.47 ($CH_2$), 75.49 et 75.72 (CH), 76.59 et 76.64 (CH), 80.77 et 81.05 (CH), 103.31 et 103.40 (CH); $\delta_c$ pour les régioisomères 3,5-*O*- **1c"** et **1c''':** 13.96 ($CH_3$), 22.13 ($CH_2$), 23.62 et 23.70 ($CH_2$), 28.92 et 28.99 (2 $CH_2$), 31.26 ($CH_2$), 34.09 et 34.51 ($CH_2$), 60.85 et 61.95 ($CH_2$), 72.42 et 73.60 ($CH_2$), 72.62 et 72.90 (CH), 73.10 et 73.99 (CH), 74.93 et 76.46 (CH), 78.36 et 79.10 (CH), 93.88 et 96.09 (CH); IR ν max: 3425 (OH), 2953 ($CH_3$), 2920 ($CH_2$), 2855, 1467, 1414, 1257, 1047; HRMS ($ESI^+$): $[M+Na]^+$ $C_{14}H_{26}NaO_5$ calculé 297.1672, mesuré 297.1670 (+1.0 ppm); HPLC (isocratique 60:40 $H_2O/CH_3CN$ + 0.1% $H_3PO_4$): $R_t$ pour les régioisomères 3,5-*O*- = 11.50 min **(1c",** 28%) et 12.93 min **(1c''',** 11%); $R_t$ pour les régioisomères 5,6-*O*- = 14.83 min **(1c,** 32%) et 16.56 **(1c',** 29%).

Exemple 1d :

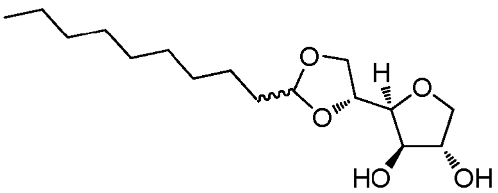

+

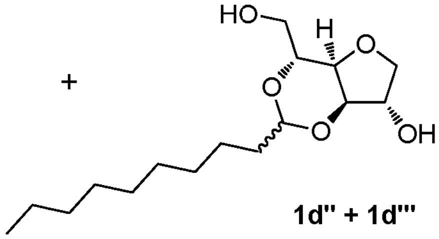

**1d + 1d'** **1d" + 1d'''**

**5,6-*O*-Decylidene-1,4-D-sorbitan 1d** et **1d'** et 3,5-*O*-decylidene-1,4-D-sorbitan **1d"** et **1d''':** Les composés ont été obtenus à partir du 1,4-D-sorbitane (1.00 g, 6 mmol) et du décanal (0.382 mL, 2 mmol) selon la procédure générale **(A).** Après réaction, le résidu est purifié par chromatographie sur colonne de gel de silice (EtOAc/cyclohexane 50:50 → 80 :20) pour donner un mélange 64:36 de régioisomères d'acétals de sorbitane en position 5,6-*O*- et 3,5-*O*- (0.098 g, 32%) sous la forme d'un solide blanc (Point de fusion = 72°C). Le produit obtenu est un mélange de régioisomères 5,6-*O*- et 3,5-*O*- d'acétals de sorbitane, chaque régioisomères étant un mélange de diastéréoisomères (35:29:25:11) comme déterminé par HPLC. RMN $^1H$ (300 MHz, $CDCl_3$) $\delta_H$ pour tous les isomères: 0.85 (3H, t, *J* = 6.9), 1.10-1.45 (14H, m), 1.47-1.70 (2H, m), 3.45 (2H, *br s,* OH protons), 3.60-4.39 (8H, m, sorbitan protons), 4.75 (t, 29%H acetal, *J* = 5.1), 4.83 (t, 11%H acetal, *J* = 4.8), 4.85 (t, 35%H acetal, *J* = 5.3), 4.97 (t, 26%H acetal, *J* = 4.8); RMN $^{13}C$ (75 MHz, $CDCl_3$) $\delta_c$ pour les régioisomères 5,6-*O*- **1d** et **1d':** 14.19 ($CH_3$), 22.76 ($CH_2$), 24.12 et 24.17 ($CH_2$), 29.40 ($CH_2$), 29.63 (3 $CH_2$), 31.97 ($CH_2$), 33.98 et 34.12 ($CH_2$), 68.17 et 68.57 ($CH_2$), 73.57 et 73.66 (CH), 73.77 et 74.13 ($CH_2$), 75.51 et 75.91 (CH), 77.30 et 77.56 (CH), 79.64 et 81.15 (CH), 104.99 et 105.14 (CH); $\delta_c$ pour les régioisomères 3,5-*O*-**1d"** et **1d''':** 14.19 ($CH_3$), 22.76 ($CH_2$), 23.84 et 24.12 ($CH_2$), 29.40 ($CH_2$), 29.63 (3 $CH_2$), 31.97 ($CH_2$), 34.19 et 34.83 ($CH_2$), 61.76 et 63.41 ($CH_2$), 72.80 et 73.14 (CH), 73.81 ($CH_2$), 75.15 et 75.34 (CH), 77.25 et 77.90 (CH), 81.37 (CH), 95.73 et 97.92 (CH); IR v max: 3433 (OH), 2918 ($CH_3$), 2851 ($CH_2$), 1739, 1123, 1080, 1048; HRMS ($ESI^+$): $[M+Na]^+$ $C_{16}H_{30}NaO_5$ calculé 325.1985, mesuré 325.1991 (-1.7 ppm); HPLC (isocratique 50:50 $H_2O/CH_3CN$ + 0.1% $H_3PO_4$): $R_t$ pour les isomères 3,5-0- = 11.97 min **(1d",** 25%) et 13.27 min **(1d''',** 11%); $R_t$ pour les régioisomères 5,6-*O*- = 15.21 min **(1d,** 35%) et 16.60 **(1d',** 29%).

Exemple 1e :

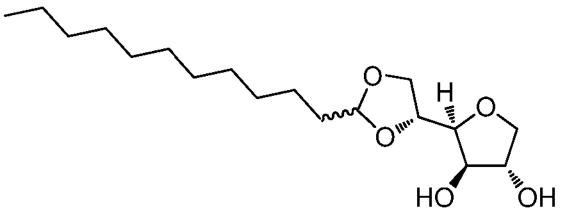

+

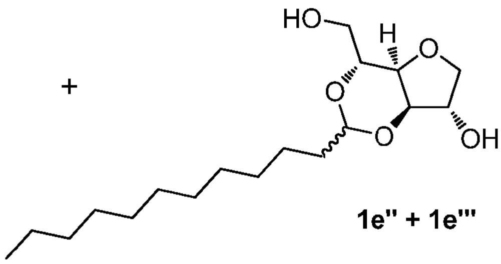

**1e + 1e'** **1e" + 1e'''**

**5,6-*O*-Dodecylidene-1,4-D-sorbitan 1e** et **1e'** et 3,5-*O*-dodecylidene-1,4-D-**sorbitan 1e"** et **1e''':** Les composés ont été obtenus à partir du 1,4-D-sorbitane (1.00 g, 6 mmol) et du dodécanal (0.450 mL, 2 mmol) selon la procédure générale **(A).** Après réaction, le résidu est purifié par chromatographie sur colonne de gel de silice (EtOAc/cyclohexane 50:50 → 70:30) pour donner un mélange 48:52 de régioisomères d'acétals de sorbitane en position 5,6-*O*- et 3,5-*O*- (0.095 g, 29%) sous la forme d'un solide blanc (Point de fusion = 82°C). Le produit obtenu est un mélange de régioisomères 5,6-*O*- et 3,5-*O*- d'acétals de sorbitane, chaque régioisomères étant un mélange de diastéréoisomères (25:23:40:12) comme déterminé par HPLC. RMN $^1H$ (300 MHz, $d_6$-DMSO) $\delta_H$ pour tous les isomères: 0.85 (3H, t, *J* = 6.9), 1.12-1.42 (18H, m), 1.43-1.59 (2H, m), 3.41-4.30 (8H, m, sorbitan protons), 4.72-4.89 (1H, m, acetal proton), 5.00-5.12 and 5.17-5.33 (2H, 2m, OH protons); RMN $^{13}C$ (75 MHz, $d_6$-DMSO) $\delta_c$ pour les régioisomères 5,6-*O*- **1e** et **1e':** 13.95 ($CH_3$), 22.15 ($CH_2$), 23.60 et 23.69 ($CH_2$), 28.79 ($CH_2$), 28.93 ($CH_2$), 29.05 ($CH_2$), 29.07 ($CH_2$), 29.08 ($CH_2$), 29.10 ($CH_2$), 31.37 ($CH_2$), 33.54 ($CH_2$), 66.59 et 66.93 ($CH_2$), 72.87 et 73.26 (CH), 73.46 ($CH_2$), 75.49 et 75.72 (CH), 76.58 et 76.63 (CH), 80.75 et 81.04 (CH), 103.29 et 103.38 (CH); $\delta_c$ pour les régioisomères 3,5-*O*- **1e"** et **1e''':** 13.95 ($CH_3$), 22.15 ($CH_2$), 23.38 et 23.60 ($CH_2$), 28.79 ($CH_2$), 28.93 ($CH_2$), 29.05 ($CH_2$), 29.07 ($CH_2$), 29.08 ($CH_2$), 29.10 ($CH_2$), 31.37 ($CH_2$), 34.10 et 34.51 ($CH_2$), 60.84 et 61.94 ($CH_2$), 72.60 et 72.95 (CH), 72.43 et 73.59 ($CH_2$), 73.17 et 73.98 (CH), 74.92 et 76.43

(CH), 78.31 et 79.07 (CH), 93.87 et 96.06 (CH); IR v max: 3412 (OH), 2917 ($CH_3$), 2849 ($CH_2$), 1468, 1418, 1256, 1082, 1050; HRMS ($ESI^+$): $[M+Na]^+$ $C_{18}H_{34}NaO_5$ calculé 353.2298, mesuré 353.2300 (-0.3 ppm); HPLC (isocratique 50:50 $H_2O/CH_3CN$ + 0.1% $H_3PO_4$): Rt pour les régioisomères 3,5-*O*- = 31.89 min **(1e",** 40%) et 35.77 min **(1e''',** 12%); $R_t$ pour les régioisomères 5,6-*O*- = 41.72 min **(1e,** 25%) et 46.18 **(1e',** 23%).

**(B)** EXEMPLE 2 : **Procédure générale pour la synthèse d'un éther de sorbitane**

Dans un autoclave de 300 mL en acier inoxydable, le mélange de régioisomères et de diastéréoisomères d'acétals de 1,4-D-sorbitan (20 mmol) est dilué dans le cyclopentylméthyléther (CPME, 200 mL) et du 5%-Pd/C (1.00 g, 5 % molaire en palladium) est ajouté. Le réacteur est fermé hermétiquement, purgé trois fois avec de l'hydrogène et l'hydrogène est introduit à une pression de 30 bars. Le mélange réactionnel est agité mécaniquement et est chauffé à 120°C pendant 15 heures. Après retour à température ambiante, la pression d'hydrogène est libérée et le mélange réactionnel est dilué dans l'éthanol absolu (EtOH, 100 mL) et filtré (filtre Millipore Durapore 0,01 μm). Le filtrat est concentré sous pression réduite pour donner le mélange de régioisomères d'éthers de sorbitane.

Exemple 2a :

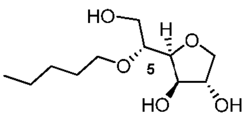

**2a**
(5-*O*-éther)

+

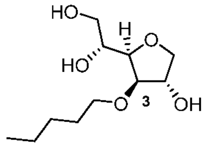

**2a'**
(3-*O*-éther)

+

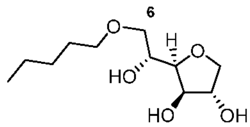

**2a"**
(6-*O*-éther)

**Pentyl-1,4-D-sorbitan 2a, 2a' et 2a":** Les composes ont été obtenus à partir du mélange 43:57 de 5,6-*O*-pentylidene-1,4-D-sorbitan **1a** et **1a'** et 3,5-O-pentylidene-1,4-D-sorbitan **1a"** et **1a'''** (0.98 g, 4.22 mmol) selon la procédure générale **(B).** Après réaction, le résidu est purifié par chromatographie sur colonne de gel de silice (EtOAc/cyclohexane 90:10 → 100: 0 puis EtOH/EtOAc 10:90) pour donner un mélange de régioisomères d'éthers de sorbitane **2a, 2a'** et **2a"** (0.686 g, 69%) sous la forme d'une pâte blanche. Le produit est un mélange 26:33:41 de 5-*O*-pentyl- **2a,** 3-*O*-pentyl- **2a'** et 6-*O*-pentyl-1,4-D-sorbitan **2a"** comme déterminé par HPLC. RMN $^1H$ (300 MHz, $d_6$-DMSO) $\delta_H$ pour tous les isomères: 0.86 (3H, t, *J* = 6.9), 1.19-1.35 (4H, m), 1.39-1.56 (2H, m), 3.22-3.99 et 4.05-4.11 (10H, m, sorbitan protons + $OCH_2$ ethers), $\delta_H$ pour l'isomère **2a:** 4.31 (1H, t, *J* = 5.8, $OH^6$), 4.84 (1H, d, *J* = 4.3, $OH^3$), 5.00 (1H, d, *J* = 2.9, $OH^2$), $\delta_H$ pour l'isomère **2a'** 14b: 4.31 (1H, t, *J* = 5.2, $OH^6$), 4.37 (1H, d, *J* = 5.4, $OH^5$), 5.06 (1H, d, *J* = 3.3, $OH^2$), $\delta_H$ pour l'isomère **2a":** 4.55 (1H, d, *J* = 5.8, $OH^5$), 4.82 (1H, d, *J* = 4.3, $OH^3$), 4.99 (1H, d, *J* = 2.8, $OH^2$); RMN $^{13}C$ (75 MHz, $d_6$-DMSO) $\delta_c$ pour l'isomère minoritaire (26%) **2a:** 14.03 ($CH_3$), 22.06 ($CH_2$), 27.88 ($CH_2$), 29.55 ($CH_2$), 62.02 ($CH_2$), 69.79 ($CH_2$), 73.15 ($CH_2$), 75.53 (CH), 76.46 (CH), 77.38 (CH), 79.29 (CH); $\delta_c$ pour l'isomère intermédiaire (33%) **2a':** 13.99 ($CH_3$), 22.03 ($CH_2$), 27.91 ($CH_2$), 29.22 ($CH_2$), 64.20 ($CH_2$), 68.72 (CH), 69.52 ($CH_2$), 73.23 (CH), 73.61 ($CH_2$), 80.10 (CH), 83.96 (CH); $\delta_c$ pour l'isomère majoritaire (41%) **2a":** 13.99 ($CH_3$), 22.02 ($CH_2$), 27.87 ($CH_2$), 28.99 ($CH_2$), 67.50 (CH), 70.60 ($CH_2$), 73.36 ($CH_2$), 73.49 ($CH_2$), 75.66 (CH), 76.38 (CH), 80.34 (CH); HRMS ($ESI^+$): $[M+Na]^+$ $C_{11}H_{22}NaO_5$ calculé 257.1359, mesuré 257.1363 (-1.4 ppm); HPLC (C18 column, isocratique 80:20 $H_2O/CH_3CN$ + 0.1% $H_3PO_4$): $R_t$ 7.20 min **(2a,** 26%), 9.25 min **(2a',** 33%) et 10.79 min **(2a",** 41%).

Exemple 2b :

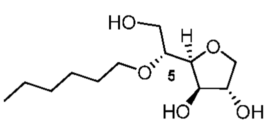

**2b**
(5-*O*-éther)

+

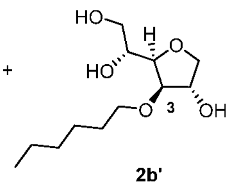

**2b'**
(3-*O*-éther)

+

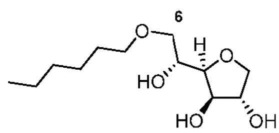

**2b"**
(6-*O*-éther)

**Hexyl-1,4-D-sorbitan 2b, 2b' et 2b":** Les composes ont été obtenus à partir du mélange 57:43 de 5,6-*O*-hexylidene-1,4-D-sorbitan **1b** et **1b'** et 3,5-*O*-hexylidene-1,4-D-sorbitan **1b"** et **1b"'** (4.92 g, 20.0 mmol) selon la procédure générale **(B).** Après réaction, le résidu est purifié par chromatographie sur colonne de gel de silice (EtOAc/cyclohexane 80:20 → 100: 0 puis EtOH/EtOAc 10:90) pour donner un mélange de régioisomères d'éthers de sorbitane **2b, 2b'** et **2b"** (3.25 g, 65%) sous la forme d'une pâte blanche. Le produit est un mélange 33:16:51 de 5-*O*-hexyl- **2b,** 3-*O*-hexyl- **2b'** et 6-*O*-hexyl-1,4-D-sorbitan **2b"** comme déterminé par HPLC. RMN $^1H$ (300 MHz, $d_6$-DMSO) $\delta_H$ pour tous les isomères: 0.86 (3H, t, *J* = 6.9), 1.16-1.36 (6H, m), 1.38-1.56 (2H, m), 3.25-4.00 et 4.05-4.11 (10H, m, sorbitan protons + $OCH_2$ ethers), $\delta_H$ pour l'isomère **2b:** 4.31 (1H, t, *J* = 5.5, $OH^6$), 4.83 (1H, d, *J* = 4.4, $OH^3$), 4.99 (1H, d, *J* = 2.9, $OH^2$), $\delta_H$ pour l'isomère **2b':** 4.31 (1H, t, *J* = 5.5, $OH^6$), 4.36 (1H, d, *J* = 5.4, $OH^5$), 5.06 (1H, d, *J* = 3.3, $OH^2$), $\delta_H$ pour l'isomère **2b":** 4.54 (1H, d, *J* = 5.8, $OH^5$), 4.81 (1H, d, *J* = 4.3, $OH^3$), 4.99 (1H, d, *J* = 2.9, $OH^2$); RMN $^{13}C$ (75 MHz, $d_6$-DMSO) $\delta_c$ pour l'isomère **2b** (33%): 14.00 ($CH_3$), 22.14 ($CH_2$), 25.36 ($CH_2$), 29.87 ($CH_2$), 31.27 ($CH_2$), 62.03 ($CH_2$), 69.84 ($CH_2$), 73.17 ($CH_2$), 75.57 (CH), 76.49 (CH), 77.40 (CH), 79.31 (CH); $\delta_c$ pour l'isomère **2b'** (16%): 13.97 ($CH_3$), 22.17 ($CH_2$), 25.34 ($CH_2$), 29.52 ($CH_2$), 31.19 ($CH_2$), 64.21 ($CH_2$), 68.74 (CH), 69.56 ($CH_2$), 73.27 (CH), 73.62 ($CH_2$), 80.11 (CH), 83.98 (CH); $\delta_c$ pour l'isomère **2b"** (51%): 13.97 ($CH_3$), 22.17 ($CH_2$), 25.40 ($CH_2$), 29.31 ($CH_2$), 31.23 ($CH_2$), 67.54 (CH), 70.65 ($CH_2$), 73.38 ($CH_2$), 73.50 ($CH_2$), 75.70 (CH), 76.40 (CH), 80.35 (CH); HRMS ($ESI^+$): $[M+Na]^+$ $C_{12}H_{24}NaO_5$ calculé 271.1516, mesuré 271.1521 (-1.7 ppm); HPLC (C18 column, isocratique 80:20 $H_2O/CH_3CN$ + 0.1% $H_3PO_4$): $R_t$ 17.49 min **(2b,** 33%), 24.45 min **(2b',** 16%) et 29.58 min **(2b",** 51%).

Exemple 2c :

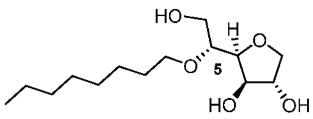
+
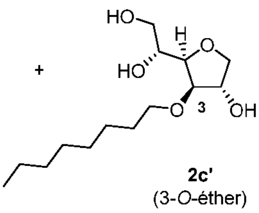
+
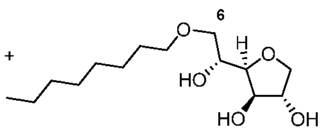

**2c** (5-*O*-éther) **2c'** (3-*O*-éther) **2c"** (6-*O*-éther)

**Octyl-1,4-D-sorbitan 2c, 2c' et 2c":** Les composes ont été obtenus à partir du mélange 61:39 de 5,6-O-octylidene-1,4-D-sorbitan **1c** et **1c'** et 3,5-O-octylidene-1,4-D-sorbitan **1c"** et **1c'"** (5.61 g, 20.4 mmol) selon la procédure générale **(B).** Après réaction, le résidu est purifié par chromatographie sur colonne de gel de silice (EtOAc/cyclohexane 80:20 → 100: 0 puis EtOH/EtOAc 10:90) pour donner un mélange de régioisomères d'éthers de sorbitane **2c, 2c'** et **2c"** (4.79 g, 85%) sous la forme d'un solide blanc. Le produit est un mélange 33:22:45 de 5-*O*-octyl- **2c,** 3-*O*-octyl- **2c'** et 6-*O*-octyl-1,4-D-sorbitan **2c"** comme determiné par HPLC. RMN $^1H$ (300 MHz, $d_6$-DMSO) $\delta_H$ pour tous lesisomères: 0.86 (3H, t, *J* = 6.8), 1.13-1.35 (10H, m), 1.36-1.55 (2H, m), 3.27-3.99 et 4.05-4.11 (10H, m, sorbitan protons + $OCH_2$ ethers), $\delta_H$ pour l'isomère **2c:** 4.31 (1H, t, *J* = 5.8, $OH^6$), 4.84 (1H, d, *J* = 4.5, $OH^3$), 5.00 (1H, d, *J* = 2.8, $OH^2$), $\delta_H$ pour l'isomère **2c':** 4.31 (1H, t, *J* = 5.2, $OH^6$), 4.37 (1H, d, *J* = 5.4, $OH^5$), 5.06 (1H, d, *J* = 3.3, $OH^2$), $\delta_H$ pour l'isomère **2c":** 4.54 (1H, d, *J* = 5.8, $OH^5$), 4.81 (1H, d, *J* = 4.3, $OH^3$), 4.99 (1H, d, *J* = 2.8, $OH^2$); RMN $^{13}C$ (75 MHz, $d_6$-DMSO): $\delta_c$ pour l'isomère **2c** (33%): 13.98 ($CH_3$), 22.13 ($CH_2$), 25.66 ($CH_2$), 28.78 ($CH_2$), 28.99 ($CH_2$), 29.89 ($CH_2$), 31.32 ($CH_2$), 62.01 ($CH_2$), 69.83 ($CH_2$), 73.15 ($CH_2$), 75.53 (CH), 76.45 (CH), 77.38 (CH), 79.29 (CH); $\delta_c$ pour l'isomère **2c'** (22%): 13.98 ($CH_3$), 22.13 ($CH_2$), 25.70 ($CH_2$), 28.75 ($CH_2$), 28.90 ($CH_2$), 29.53 ($CH_2$), 31.30 ($CH_2$), 64.18 ($CH_2$), 68.71 (CH), 69.52 ($CH_2$), 73.23 (CH), 73.60 ($CH_2$), 80.08 (CH), 83.95 (CH); $\delta_c$ pour l'isomère **2c"** (45%): 13.98 ($CH_3$), 22.13 ($CH_2$), 25.70 ($CH_2$), 28.75 ($CH_2$), 28.93 ($CH_2$), 29.32 ($CH_2$), 31.30 ($CH_2$), 67.49 (CH), 70.61 ($CH_2$), 73.36 ($CH_2$), 73.49 ($CH_2$), 75.66 (CH), 76.37 (CH), 80.34 (CH); HRMS ($ESI^+$): $[M+Na]^+$ $C_{14}H_{28}NaO_5$ calculé 299.1829, mesuré 299.1832 (-1.2 ppm); HPLC (C18 column, isocratique 60:40 $H_2O/CH_3CN$ + 0.1% $H_3PO_4$): $R_t$ 8.79 min **(2c,** 33%), 9.80 min **(2c',** 22%) et 11.77 min **(2c",** 45%).

Exemple 2d :

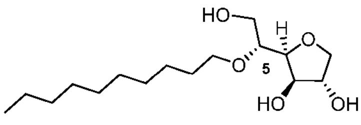

**2d**
(5-*O*-éther)

+

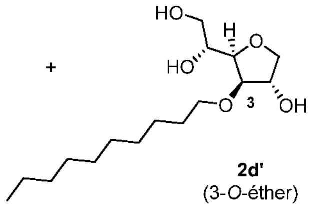

**2d'**
(3-*O*-éther)

+

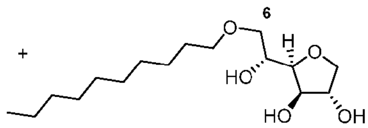

**2d"**
(6-*O*-éther)

**Décyl-1,4-d-sorbitan 2d, 2d'** et **2d":** Les composes ont été obtenus à partir du mélange 64:36 de 5,6-*O*-décylidene-1,4-D-sorbitan **1d** et **1d'** et 3,5-*O*-décylidene-1,4-D-sorbitan **1d"** et **1d'''** (6.12 g, 20.2 mmol) selon la procédure générale **(B).** Après réaction, le résidu est purifié par chromatographie sur colonne de gel de silice (EtOAc/cyclohexane 70:30 → 100: 0 puis EtOH/EtOAc 10:90) pour donner un mélange de régioisomères d'éthers de sorbitane **2d, 2d'** et **2d"** (3.66 g, 59%) sous la forme d'un solide blanc. Le produit est un mélange 32:16:52 de 5-*O*-décyl- **2d,** 3-*O*-décyl-**2d'** et 6-*O*-décyl-1,4-D-sorbitan **2d"** comme déterminé par HPLC. RMN $^1H$ (300 MHz, $d_6$-DMSO) $\delta_H$ pour tous les isomères: 0.85 (3H, t, *J* = 6.9), 1.14-1.35 (14H, m), 1.37-1.55 (2H, m), 3.25-3.98 et 4.05-4.11 (10H, m, sorbitan protons + $OCH_2$ ethers), $\delta_H$ pour l'isomère **2d:** 4.31 (1H, t, *J* = 5.4, $OH^6$), 4.82 (1H, d, *J* = 4.3, $OH^3$), 4.99 (1H, d, *J* = 2.9, $OH^2$), $\delta_H$ pour l'isomère **2d':** 4.31 (1H, t, *J* = 5.4, $OH^6$), 4.35 (1H, d, *J* = 5.5, $OH^5$), 5.06 (1H, d, *J* = 3.3, $OH^2$), $\delta_H$ pour l'isomère **2d":** 4.53 (1H, d, *J* = 5.8, $OH^5$), 4.80 (1H, d, *J* = 4.3, $OH^3$), 4.98 (1H, d, *J* = 1.9, $OH^2$); RMN $^{13}C$ (75 MHz, $d_6$-DMSO) $\delta_c$ pour l'isomère **2d** (32%): 13.98 ($CH_3$), 22.16 ($CH_2$), 25.69 ($CH_2$), 28.79 ($CH_2$), 29.07 ($CH_2$), 29.10 ($CH_2$), 29.17 ($CH_2$), 29.92 ($CH_2$), 31.37 ($CH_2$), 62.01 ($CH_2$), 69.84 ($CH_2$), 73.16 ($CH_2$), 75.56 (CH), 76.48 (CH), 77.41 (CH), 79.30 (CH); $\delta_c$ pour l'isomère **2d'** (16%): 13.98 ($CH_3$), 22.16 ($CH_2$), 25.72 ($CH_2$), 28.79 ($CH_2$), 28.98 ($CH_2$), 29.07 ($CH_2$), 29.12 ($CH_2$), 29.57 ($CH_2$), 31.37 ($CH_2$), 64.18 ($CH_2$), 68.72 (CH), 69.55 ($CH_2$), 73.27 (CH), 73.60 ($CH_2$), 80.08 (CH), 83.96 (CH); $\delta_c$ pour l'isomère **2d"** (52%): 13.98 ($CH_3$), 22.16 ($CH_2$), 25.72 ($CH_2$), 28.79 ($CH_2$), 29.01 ($CH_2$), 29.07 ($CH_2$), 29.14 ($CH_2$), 29.35 ($CH_2$), 31.37 ($CH_2$), 67.53 (CH), 70.64 ($CH_2$), 73.37 ($CH_2$), 73.50 ($CH_2$), 75.69 (CH), 76.40 (CH), 80.35 (CH); HRMS (ESI+): $[M+Na]^+$ $C_{16}H_{32}NaO_5$ calculé 327.2142, mesuré 327.2135 (+2.1 ppm); HPLC (C18 column, isocratique 50:50 $H_2O/CH_3CN$ + 0.1% $H_3PO_4$): $R_t$ 9.03 min **(2d,** 32%), 9.67 min **(2d',** 16%) et 11.61 min **(2d",** 52%).

Exemple 2e :

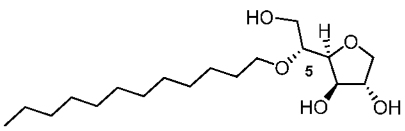

**2e**
(5-*O*-éther)

+

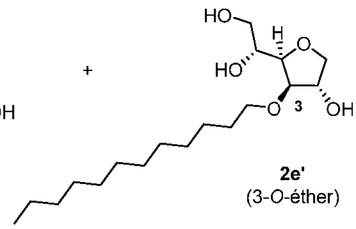

**2e'**
(3-*O*-éther)

+

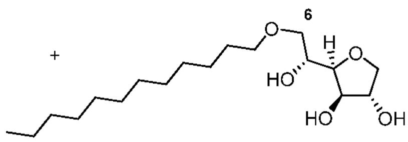

**2e"**
(6-*O*-éther)

**Dodécyl-1,4-d-sorbitan 2e, 2e' et 2e"** : Les composes ont été obtenus à partir du mélange 48:52 de 5,6-*O*-dodécylidene-1,4-D-sorbitan **1e** et **1e'** et 3,5-*O*-dodécylidene-1,4-D-sorbitan **1e"** et **1e'''** (1.29 g, 3.92 mmol) selon la procédure générale **(B).** Après réaction, le résidu est purifié par chromatographie sur colonne de gel de silice (EtOAc/cyclohexane 70:30 → 100: 0 puis EtOH/EtOAc 10:90) pour donner un mélange de régioisomères d'éthers de sorbitane **2e, 2e'** et **2e"** (0.72 g, 55%) sous la forme d'une huile incolore. Le produit est un mélange 27:33:40 de 5-*O*-dodécyl-**2e,** 3-*O*-dodécyl-**2e'** et 6-*O*-dodécyl-1,4-D-sorbitan **2e"** comme déterminé par HPLC. RMN $^1H$ (300 MHz, $d_6$-DMSO) $\delta_H$ pour tous les isomères: 0.85 (3H, t, *J* = 6.9), 1.16-1.34 (18H, m), 1.38-1.54 (2H, m), 3.26-3.98 et 4.05-4.11 (10H, m, sorbitan protons + $OCH_2$ ethers), $\delta_H$ pour l'isomère **2e:** 4.32 (1H, t, *J* = 5.5, $OH^6$), 4.84 (1H, d, *J* = 3.7, $OH^3$), 5.00 (1H, d, *J* = 2.8, $OH^2$), $\delta_H$ pour l'isomère **2e':** 4.32 (1H, t, *J* = 5.5, $OH^6$), 4.37 (1H, d, *J* = 5.4, $OH^5$), 5.06 (1H, d, *J* = 3.3, $OH^2$), $\delta_H$ pour l'isomère **2e":** 4.55 (1H, d, *J* = 5.8, $OH^5$), 4.82 (1H, d, *J* = 4.1, $OH^3$), 4.99 (1H, d, *J* = 2.1, $OH^2$); RMN $^{13}C$ (75 MHz, $d_6$-DMSO) $\delta_c$ pour l'isomère **2e** (27%): 13.97 ($CH_3$), 22.11 ($CH_2$), 25.64 ($CH_2$), 28.74 ($CH_2$), 29.05 (3 $CH_2$), 29.08 (2 $CH_2$), 29.88 ($CH_2$), 31.32 ($CH_2$), 62.00 ($CH_2$), 69.81 ($CH_2$), 73.14 ($CH_2$), 75.52 (CH), 76.44 (CH), 77.38 (CH), 79.27 (CH); $\delta_c$ pour l'isomère **2e'** (33%): 13.97 ($CH_3$), 22.11 ($CH_2$), 25.68 ($CH_2$), 28.74 ($CH_2$), 29.05 (3 $CH_2$), 29.08 (2 $CH_2$), 29.52 ($CH_2$), 31.32 ($CH_2$), 64.16 ($CH_2$), 68.69 (CH), 69.51 ($CH_2$), 73.22 (CH), 73.58 ($CH_2$), 80.06 (CH), 83.93 (CH); $\delta_c$ pour l'isomère **2e"** (40%): 13.97 ($CH_3$), 22.11 ($CH_2$), 25.68 ($CH_2$), 28.74 ($CH_2$), 28.92 ($CH_2$), 28.96 ($CH_2$), 29.05 (2 $CH_2$), 29.08 ($CH_2$), 29.31 ($CH_2$), 31.32 ($CH_2$), 67.47 (CH), 70.59 ($CH_2$), 73.35 ($CH_2$), 73.48 ($CH_2$), 75.63 (CH), 76.35 (CH), 80.34 (CH); HRMS (ESI+): $[M+Na]^+$ $C_{18}H_{36}NaO_5$ calculé 355.2455, trouvé 355.2458 (-0.9 ppm); HPLC (C18 column, isocratique 50:50 $H_2O/CH_3CN$ + 0.1% $H_3PO_4$): $R_t$ 22.65 min **(2e,** 27%), 25.04 min **(2e',** 33%) et 30.81 min **(2e",** 40%).

EXEMPLE 3 : **Procédure « one-pot » pour la synthèse d'un éther de sorbitane**

*Synthèse « one-pot » d'éthers de sorbitane à partir du 1,4-sorbitane :*

Dans un ballon à fond rond de 100 mL, du 1,4-sorbitane (10 g, 62 mmol) est dissout dans du CPME sec (30 mL) en présence de $Na_2SO_4$ (6,5 g, 50 mmol), sous une atmosphère d'argon. Du valéraldehyde (3,3 mL, 31 mmol) est ajouté, goutte à goutte, suivi d'Amberlyst 15 (530 mg, 20 %m en valéraldehyde). Le mélange est chauffé jusqu'à 80°C sous agitation magnétique. Après 3 heures, le mélange chaud est filtré, lavé avec du CPME (2 x 25 mL) et le filtrat est concentré sous pression réduite. Sans purification additionnelle, le mélange est dilué dans du CPME (300 mL), séché sur du $MgSO_4$ et filtré. Le filtrat est introduit dans un autoclave en inox de 500 mL, et du 5%-Pd/C (3,3 mg) est ajouté. Le réacteur est bien fermé, purgé trois fois avec de l'hydrogène, avant que de l'hydrogène soit introduit sous pression (30 bar). Le système est chauffé à 120°C et agité pendant 15 heures. Après avoir été refroidi jusqu'à la température ambiante, l'hydrogène sous pression est relâché, le mélange réactionnel est dissout dans de l'éthanol absolu (250 mL) et filtré (Filtre Millipore Durapore 0,01 micron). Le filtrat est évaporé sous pression réduite et le résidu (5,8 g) est purifié par chromatographie flash (EtOAc/cyclohexane 90:10 à 100:0, puis EtOH/EtOAc 10:90). Ainsi un mélange d'éthers de pentyl-(1,4)-sorbitane (3,97 g, 56%) a été obtenu sous forme d'une huile incolore (pureté >98% par RMN 1H).

EXEMPLE 4 : **Mesure des propriétés bactériostatiques de dérivés acétals et éthers de sorbitane sur des bactéries à Gram positifs**

Les propriétés bactériostatiques des dérivés sont évaluées par la mesure de leur concentration minimale inhibitrice (CMI) vis-à-vis des bactéries testées. Une telle mesure est réalisée par une la méthode de microdilution réalisée en microplaque à 96 puits selon les conditions définies ci-dessous.

*Les bactéries testées :*

Les croissances minimales inhibitrices (CMI) sont réalisées sur des souches bactériennes Gram positif selon les recommandations du « Clinical Laboratory Standards Institute » (Clinical-Laboratory-Standards-Institute, 6th ed. Approved standard M100-S17. CLSI, Wayne, PA, 2007).

Les bactéries Gram positif étudiées sont les suivantes : *L. monocytogenes* (CIP 103575), *E. faecalis* (ATCC® 29212™) et S. *aureus* (ATCC®292213™).

*Préparation de l'inoculum :*

Les cultures étudiées, fraîchement isolées (après incubation sur une gélose de sang à 37°C pendant 18h), sont reprises dans de l'eau stérile (10 mL) jusqu'à obtenir une suspension à 0,5 Mac Farland (Mc) soit 1 à $2 \times 10^8$ UFC (bactérie)/$cm^3$. La suspension bactérienne a ensuite été diluée afin d'obtenir une concentration finale de $5 \times 10^5$ UFC/$cm^3$.

*Préparation des plaques multipuits pour la lecture de la CMI :*

Chaque puits contient une quantité identique de milieu Mueller-Hinton (milieu riche permettant la culture des bactéries) et de bactérie de $5 \times 10^5$ UFC/$cm^3$ finale.

Les composés d'intérêt à tester sont solubilisés dans 2,5%m d'éthanol avant d'être dilués à différentes concentrations de deux en deux.

Sur la plaque multipuits, une première série a été prévue comprenant le milieu de culture sans le composé d'intérêt à tester. Il correspondant au témoin de croissance (puits témoins). Ces témoins servent de référence pour comparer la croissance bactérienne avec celles des puits suivants comprenant différentes concentrations du composé d'intérêt à tester. La seconde série de puits comprend la solution mère du composé d'intérêt à tester pour une concentration dans le puits de 4 mM. Chaque série de puits a été diluée de deux en deux jusqu'à la dernière série pour une concentration finale de 0,003 mM. Chaque concentration est dupliquée au sein de la même plaque. La plaque est incubée 18h à 37°C. La lecture après incubation montre un trouble dans les puits témoins (révélateur d'une croissance bactérienne). En cas d'activité antibactérienne, la croissance bactérienne est inhibée ce qui se traduit par l'absence d'apparition de trouble ou culot bactérien. L'inhibition de cette croissance bactérienne par le composé testé peut correspondre soit à une activité bactériostatique de la molécule (inhibe la croissance bactérienne), soit à une activité bactéricide de la molécule (provoque la mort de la bactérie).

*Dénombrement bactérien* :

Afin de déterminer si les agents testés sont bactéricides, la concentration minimale bactéricide (CMB) est déterminée. La CMB correspond à la concentration laissant un nombre de survivant bactérien < 4 Log. Pour cela un dénombrement bactérien est effectué à partir des puits clairs ou sans culot bactérien (C≤CMI). Pour ce faire, une dilution au $\frac{1}{100}$ a été effectuée avec les deux puits de même concentration avant ensemencement sur une gélose de sang à l'aide de la technique Spirale. Après incubation de 24h à 37°C, le dénombrement visuel a permis de déterminer, la concentration minimale à partir de laquelle il n'y a plus croissance bactérienne.

Les tests ont été réalisés sur les bactéries Gram positif avec les dérivés de sorbitane. Les solutions de composés à tester sont diluées dans l'éthanol à une concentration en solvant qui n'agit pas sur la croissance bactérienne (2,5 %m). Les solutions après stérilisation sont diluées dans l'eau. Les résultats des tests antimicrobiens obtenus sur les 3 souches bactériennes *L. monocytogenes* (CIP 103575), *E. faecalis* (ATCC® 29212™) et *S. aureus* (ATCC® 292213 ™) sont résumés dans le Tableau 1.

**Tableau 1. Résultats antimicrobiens pour les dérivés du sorbitane sur les Gram positif :** *concentration Minimale Inhibitrice (CMI) en mmol/L*

| | | Fonction | | | | | |
|---|---|---|---|---|---|---|---|
| Entrée | Chaîne alkyle | Acétal (Ac) | | | Ether (Eth) | | |
| | | *L. monocyto genes* | *S. aureus.* | *E. faecalis* | *L. monocyto genes* | *S. aureus.* | *E. faecalis* |
| 1 | C5 | 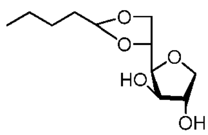 Mélange isomérique en position (5,6):(3,5) : 36 :64 | | | 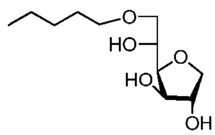 Mélange isomérique en position 3 :5 :6 : 33 : 26 :41 | | |
| | | >4 | >4 | >4 | >4 | >4 | >4 |
| 2 | C6 | 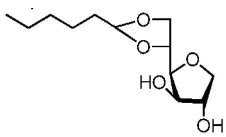 Mélange isomérique en position (5,6):(3,5) : 57 :43 | | | 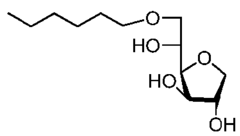 Mélange isomérique en position 3 :5 :6 : 16 : 33 :51 | | |
| | | *>4* | *>4* | *>4* | *>4* | *>4* | *>4* |
| 3 | C8 | 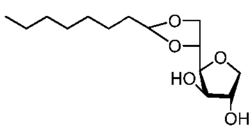 Mélange isomérique en position (5,6):(3,5) : 61 :39 | | | 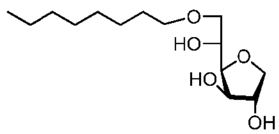 Mélange isomérique en position 3 :5 :6 : 22 : 33 :45 | | |
| | | *>4* | *>4* | *>4* | *>4* | *>4* | *>4* |
| 4 | C10 | 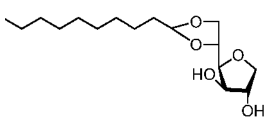 Mélange isomérique en position (5,6):(3,5) : 64 :36 | | | 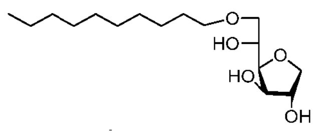 Mélange isomérique en position 3 :5 :6 : 16 : 32 :52 | | |
| | | *>4* | *>4* | *>4* | *>4* | *>4* | *>4* |

(suite)

| | | Fonction | | | | | |
|---|---|---|---|---|---|---|---|
| Entrée | Chaîne alkyle | Acétal (Ac) | | | Ether (Eth) | | |
| | | *L. monocytogenes* | *S. aureus.* | *E. faecalis* | *L. monocytogenes* | *S. aureus.* | *E. faecalis* |
| 5 | C12 | 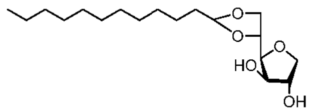 Mélange isomérique en position (5,6):(3,5) : 50 :50 | | | 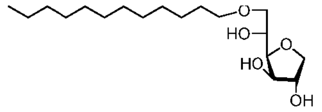 Mélange isomérique en position 3 :5 :6 : 33 : 27 :40 | | |
| | | *0,03* | *0,12* | *0,03* | *0,12* | *0,12* | *0,12* |

D'après l'observation des microplaques à 96 puits, les éthers et acétals de sorbitane avec des chaînes aliphatiques inférieures ou égales à 10 carbones ne présentent pas de propriétés antimicrobiennes car tous les puits contiennent un trouble ou un culot bactérien. L'unique inhibition bactérienne est observée pour les composés dérivés du dodécyle (entrée 5).

En effet, avec des concentrations inférieures à 12 mM, l'acétal et l'éther en C12 du sorbitane inhibent les souches bactériennes étudiées.

EXEMPLE 5 : **Propriété bactéricide des dérivés acétals ou éthers de sorbitane sur des bactéries à Gram positifs**

Afin de déterminer l'effet bactéricide des composés présentant des propriétés bactériostatiques, les puits ne présentant plus de trouble ont été réensemencés sur gélose. Les résultats obtenus après incubation d'une nuit sont présentés dans le Tableau 2.

**Tableau 2. Résultats antimicrobiens pour les dérivés du sorbitane sur les Gram positif** : Concentration Minimale Inhibitrice (CMI) en mmol/L, Concentration Minimale Bactéricide (CMB) en mmol/L (en italique)

| Entrée | Bactérie | Sorbitane (Sorb) | |
|---|---|---|---|
| | | AcC12 | EthC12 |
| 1 | *L. monocytogenes* | *0,03* | *0,12* |
| 2 | *S. aureus* | 0,12 | *0,12* |
| 3 | *E. faecalis* | *0,03* | *0,12* |

Concernant les dérivés du sorbitane, uniquement les composés contenants des chaînes à 12 carbones et présentant une inhibition bactérienne ont été analysés. L'acétal dodécylidène de sorbitane s'est révélé être un composé bactéricide des souches L. *monocytogenes* et *E. faecatis.à* 0,03 mM et bactériostatique *S. aureus* à 0,12 mM. Afin de confirmer que les propriétés mesurées des acétals sont bien celles du composé amphiphile et non de ses produits d'hydrolyse, les propriétés du dodécanal ont été testées sur les différentes souches bactériennes et aucune activité antimicrobienne n'a été observée à des concentrations inférieures ou égales à 4 mM. Ainsi, l'acétal en C12 du sorbitane est actif en tant que tel et cette activité ne provient pas de l'aldéhyde correspondant. Le mélange d'éthers dodécyl sorbitane possède une CMB de 0,12 mM pour toutes les souches Gram positif testées.

On peut donc conclure que les acétals et éthers de sorbitane en C12, même sous la forme de mélange de régioisomères et diastéréoisomères, présentent des propriétés antimicrobiennes et bactéricides très intéressantes.

Ces résultats montrent que les dérivés de sorbitane peuvent présenter une nouvelle gamme de produits bactériostatiques et bactéricides biosourcés est très actives.

*EXEMPLE 6 :* **Evaluation des propriétés tensioactives et antimicrobiennes**

Les propriétés physico-chimiques et antimicrobiennes des meilleurs produits synthétisés ont pu être testées. Ces analyses mettent en évidence les différents profils de tensioactifs, ainsi que les valeurs des Concentrations Minimales Inhibitrices (CMI) de chaque composé sur des bactéries Gram positif. Les meilleurs résultats tensioactifs et antimicrobiens

sont comparés dans le Tableau 3.

**Tableau 3. Résultats comparatifs entre les concentrations micellaires critiques (CMC) et les concentrations minimales inhibitrices (CMI) en (mmol/L) sur les produits d'intérêt** : *concentration Minimale Inhibitrice (CMI) en mmol/L*

| Entrée | Composé | CMC (mM) | CMI (mM) | | |
|---|---|---|---|---|---|
| | | | *L. monocytogenes* | *S. aureus.* | *E. faecalis* |
| 1 | C12-Ac-Sorb 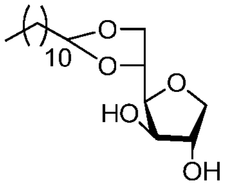 | 0,034 | 0,03 | 0,12 | 0,03 |
| 1 | C12-Eth-Sorb 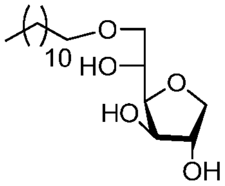 | 0,091 | 0,12 | 0,12 | 0,12 |

Pour le dodécylidène sorbitane (entrée 1), la valeur des CMC se trouve dans la gamme de CMI. L'éther dodécyl sorbitane possède, quant à lui, une CMC légèrement plus faible (0,09 mM) que sa CMI (0,12 mM) mais ces concentrations sont tout de même relativement proches (entrée 2).

*<u>EXEMPLE 7</u>* **Tests comparatifs avec des composés connus de l'art antérieur**

L'activité des dérivés de sorbitane a été comparée avec celle de composés de structures proches ou d'un composé commercial comme la monolaurine (ML) dans le tableau ci-dessous.

**Tableau 4. Résultats comparatifs entre des produits de référence (ML) et les acétals et éthers de sorbitane** : *concentration Minimale Inhibitrice (CMI) en mmol/L*

| | Composés connus de l'art antérieur | Composés testés | |
|---|---|---|---|
| Bactéries | Monolaurine (ML) | C12-Eth-Sorb | C12-Ac-Sorb |
| | | 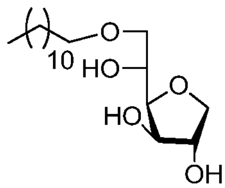 Mélange isomérique en position 3 : 5 :6 : 33 :27 :40 | 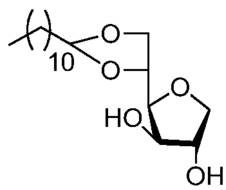 Mélange isomérique en position 3,5- et 5,6- : 50 : 50 |
| *L. monocytogenes.* | 0,04 | 0,12 | 0,03 |
| *S. aureus* | 0,04 | 0,12 | 0,12 |
| *E.. faecalis* | nd | 0,12 | 0,03 |

*Les résultats obtenus démontrent que les dérivés selon l'invention sont tout aussi efficaces que la monolaurine (ML) puisque la différence de CMI obtenue entre les mélanges d'acétals (C12AcSorb) ou d'éthers C12 de sucre*

*(C12EthSorb) et la monolaurine est faible.*

*Cependant, la stabilité des fonctions éthers en milieu biologique étant plus élevée que les esters (sensibles aux estérases), les composés comprenant une fonction éther auront donc une activité prolongée dans le temps ce qui fait de ces dérivés des composés particulièrement avantageux.*

EXEMPLE 8 : **Mesure des propriétés bactériostatiques d'un éther de sorbitane en C12 sur des bactéries à Gram positifs**

Les meilleurs résultats ayant été observés avec des composés ayant un groupement alkyl en C12, des essais ont été effectués sur un plus large panel de souches à gram positif avec *un mélange d'éthers de sorbitane* tel qu'obtenu selon les exemples précédents.

- *Préparation de l'inoculum :*

Les cultures étudiées, fraîchement isolées (après incubation sur une gélose de sang à 37°C pendant 18h), sont reprises dans de l'eau stérile (10 mL) jusqu'à obtenir une suspension à 0,5 Mac Farland (Mc) soit à 1 à 2 $\times$ $10^8$ UFC (bactérie)/$cm^3$. La suspension bactérienne a ensuite été diluée afin d'obtenir une concentration finale de 1 $\times$ $10^6$ UFC/$cm^3$.

- *Préparation des plaques multipuits pour la lecture de la CMI:*

Chaque puits contient une quantité identique de milieu Mueller-Hinton (milieu riche permettant la culture des bactéries) et de bactérie de 0.5 $\times$ $10^6$ UFC/$cm^3$ finale.

Les composés d'intérêt à tester sont solubilisés dans l'éthanol ou DMSO à 25 mg/mL avant d'être dilués à différentes concentrations de deux en deux. Sur la plaque multi-puits, une première série a été prévue comprenant le milieu de culture sans le composé d'intérêt à tester. Il correspond au témoin de croissance (puits témoins). Ces témoins servent de référence pour comparer la croissance bactérienne avec celles des puits suivants comprenant différentes concentrations du composé d'intérêt à tester. La seconde série de puits comprend la solution mère du composé d'intérêt à tester pour une concentration dans le puit de 256 mg/L (7 mM). Chaque série de puits a été diluée de deux en deux jusqu'à la dernière série pour une concentration finale de 0.25 mg/L (0,0007 mM). Chaque concentration est dupliquée au sein de la même plaque. La plaque est incubée 18h à 37°C. La lecture après incubation montre un trouble dans les puits témoins (révélateur d'une croissance bactérienne). En cas d'activité antibactérienne, la croissance bactérienne est inhibée ce qui se traduit par l'absence d'apparition de trouble ou culot bactérien.

Les croissances minimales inhibitrices (CMI) sont réalisées sur des souches bactériennes Gram positif selon les recommandations du « Clinical Laboratory Standards Institute » (Clinical-Laboratory-Standards-Institute, 6th ed. Approved standard M100-S17. CLSI, Wayne, PA, 2007). Les souches cliniques ont été isolées à l'hospice de Lyon.

**Les bactéries Gram positif étudiées sont les suivantes** :

- **Staphylocoques *S*. aureus;** ATCC®29213™, ATCC 25923,
  Souches de Staphylocoques *S*. aureus resistantes à la methiciline (Lac-Deleo USA 300), (MU 3), (HT 2004-0012), LY 199-0053, (HT 2002-0417), (HT 2006-1004),
  Souches de Staphylocoques *S*. aureus resistantes à la daptomycine (ST 2015-0188), (ST 2014 1288).

- **Enterocoques:** *E. faecalis* (ATCC® 29212™), souches cliniques d'enterocoques *E. faecalis* isolées d'urines : la souche 015206179901 (ci-après 9901), la souche 015205261801 (ci-après 1801)

- **Enterocoques:** *E. faecium* (CIP 103510), souches cliniques *d'*Enterocoques *E. faecium:* Van A 0151850763 (ci-après Van A); la souche 015 205731401 (ci-après 1401),

- **Listeria:** *L. monocytogenes* (CIP 103575), souche cliniques isolée d'hémoculture (015189074801, LM1), souche isolée du liquide céphalo-rachidien (015170199001, LM2), souche clinique isolée d'hémoculture (015181840701, LM3).

- *Préparation de l'inoculum :*

Les cultures étudiées, fraîchement isolées (après incubation sur une gélose de sang à 37°C pendant 18h), sont reprises dans de l'eau stérile (10 mL) jusqu'à obtenir une suspension à 0,5 Mac Farland (Mc) soit à $10^8$ UFC (bactérie)/$cm^3$. La suspension bactérienne a ensuite été diluée afin d'obtenir une concentration finale de $10^6$ UFC/$cm^3$

- *Résultats pour les souches du Genre des Staphylococcus*

***Tableau 5**. Résultats antimicrobiens pour un éther de sorbitane sur différentes souches de Staphylocoques Aureus : Concentration Minimale Inhibitrice (CMI) en mg/L.*

| | *Staphylococcus* | | | | | | | | | |
|---|---|---|---|---|---|---|---|---|---|---|
| | ATCC 25923 | ATCC 29213 | USA 300 | MU 3 | HT 2004-0012 | LY 199-0053 | HT 2002-0417 | HT 2006-1004 | ST 2015 0188 | ST 2014 1288 |
| C12-Eth-Sorb 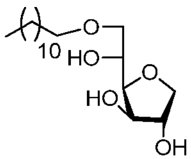 | 32 | 32 | 32 | 64 | 32 | 32 | 32 | 32 | 64 | 64 |

D'après l'observation des microplaques à 96 puits, l'éther de sorbitane en C12 (C12-Eth-Sorb) est actif contre les souches de *staphylocoques* testées (32 < CMI < 64 mg/L).

- *Résultats pour les souches du Genre des Enterococcus*

***Tableau 6.*** *Résultats antimicrobiens pour un éther de sorbitane sur différentes souches d'entérocoques. Concentration Minimale Inhibitrice (CMI) en mg/L*

| | **Enterococcus** | | | | | |
|---|---|---|---|---|---|---|
| | **ATCC29 212** | **Van A** | **CIP 103510** | **1401** | **9901** | **1801** |
| C12-Eth-Sorb 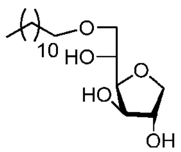 | 8 | 16 | 16 | 8 | 16 | 8 |

On observe une bonne activité antibactérienne de l'éther en C12 de sorbitane pour toutes les souches d'Enterocoques testées 8 < CMI < 16 mg/L.

- *Résultats pour les souches du genre des Listeria*

***Tableau 7.*** *Résultats antimicrobiens pour un éther de sorbitane sur différentes souches de Listeria concentration Minimale Inhibitrice (CMI) en mg/L.*

| | **Listeria** | | | |
|---|---|---|---|---|
| | CIP 103575 | LM1 | LM2 | LM3 |
| C12-Eth-Sorb 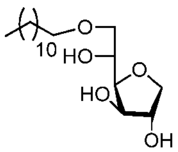 | 32 | 16 | 32 | 32 |

On observe un bonne activité antibactérienne de l'éther en C12 de sorbitane sur toutes les souches de Listeria testées 16 < CMI < 32 mg/L.

## Revendications

1. Composition bactéricide ou bactériostatique pour son utilisation dans le traitement ou la prévention des infections bactériennes par des bactéries à Gram positif, **caractérisée en ce qu'**elle comprend un mélange de régioisomères et/ou de diastéréoisomères d'un acétal d'alkyle ou un éther d'alkyle de sorbitane, ou un sel pharmaceutiquement acceptable, ledit groupe alkyle comprenant entre 11 à 18 atomes de carbone, ledit radical acétal alkyle ou éther alkyle étant en position 2-*O*-, 3-*O*-, 5-0- et/ou 6-*O*-.

2. Composition pour son utilisation selon la revendication **1**, **caractérisée en ce que** le groupe alkyle comprend 11 à 13 atomes de carbone.

3. Composition pour son utilisation selon l'une ou l'autre des revendications **1** et **2**, **caractérisée en ce que** ledit radical acétal alkyle est en position 2,3-*O*- ; 3,5-*O*- ou 5,6-*O*-.

**4.** Composition pour son utilisation selon l'une quelconque des revendications **1** à **3**, **caractérisée en ce que** les bactéries à Gram positif sont des bactéries de l'embranchement des Firmicutes, typiquement de la classe des Bacilli notamment choisies parmi les bactéries de l'ordre des Lactobacillales ou des Bacillales.

**5.** Composition pour son utilisation selon la revendication **4**, **caractérisée en ce que** les bactéries à Gram positif sont des bactéries de l'ordre des Bacillales choisies parmi la famille des Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea et Turicibacteraceae.

**6.** Composition pour son utilisation selon l'une ou l'autre des revendications **4** et **5**, **caractérisée en ce que** les bactéries à Gram positif sont des bactéries de la famille des Listeriaceae telle qu'une bactérie du genre des *Brochothrix* ou des *Listeria* typiquement, choisies parmi *L. fleischmannii, L grayi, L. innocua, L. ivanovii, L. marthii, L. monocytogenes, L. rocourtiae, L. seeligeri, L. weihenstephanensis* et *L. welshimeri.*

**7.** Composition pour son utilisation selon l'une ou l'autre des revendications **4** et **5**, **caractérisée en ce que** les bactéries à Gram positif sont des bactéries de la famille des Staphylococcaceae choisies parmi les bactéries du genre des *Staphylococcus, Gemella, Jeotgalicoccus, Macrococcus, Salinicoccus* et *Nosocomiicoccus.*

**8.** Composition pour son utilisation selon la revendication **7**, **caractérisée en ce que** les bactéries à Gram positif sont des bactéries du genre des *Staphylococcus* choisies parmi *S. arlettae, S. agnetis, S. aureus, S. auricularis, S. capitis, S. caprae, S. carnosus, S. caseolyticus, S. chromogenes, S. cohnii, S. condi menti, S. delphini, S. devriesei, S. epider midis, S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri* et S. *xylosus.*

**9.** Composition pour son utilisation selon la revendication **4**, **caractérisée en ce que** les bactéries à Gram positif sont des Lactobacillales choisies parmi une famille des Aerococcaceae, Carnobacteriaceae, Enterococcaceae, Lactobacillaceae, Leuconostocaceae et Streptococcaceae.

**10.** Composition pour son utilisation selon la revendication **9**, **caractérisée en ce que** les bactéries à Gram positif sont des bactéries de la famille des Enterococcaceae choisies parmi les bactéries du genre des *Bavariicoccus, Catellicoccus, Enterococcus, Melissococcus, Pilibacter, Tetragenococcus, Vagococcus.*

**11.** Composition pour son utilisation selon la revendication **10**, **caractérisée en ce que** les bactéries à Gram positif sont des bactéries du genre des *Enterococcus* choisies parmi *E. malodoratus, E. avium, E. dur ans, E. faecalis, E. faecium, E. gallinarum, E. hirae, E. solitarius,* préférentiellement, *E. avium, E. durans, E. faecalis* et *E. faecium.*

**12.** Composition pour son utilisation selon l'une quelconque des revendications **1** à **11**, ladite composition étant sous forme de composition pharmaceutique, vétérinaire, produit d'hygiène ou dermatologique à usage externe.

**13.** Composition pour son utilisation selon l'une quelconque des revendications **1** à **12,** dans laquelle l'infection par des bactéries à Gram positif est une infection de la peau ou des muqueuses, préférentiellement une infection choisie parmi une folliculite, un abcès, un panaris, un furoncle, un impétigo, une infection interdigitales, un anthrax (anthrax staphylococcique), une cellulite, une surinfection de plaies, un otitis sinusitis, une hydradénite, une mastite infectieuse, une infection post-traumatique de la peau et une infection de la peau brûlée.

**14.** Méthode de désinfection ou de prévention de la colonisation bactérienne par des bactéries à Gram positif d'un substrat comprenant la mise en contact du substrat avec une composition ladite composition comprenant un mélange de régioisomères et/ou de diastéréoisomères d'un acétal d'alkyle ou un éther d'alkyle de sorbitane, ou un sel pharmaceutiquement acceptable, ledit groupe alkyle comprenant entre 11 à 18 atomes de carbone, ledit radical acétal alkyle ou éther alkyle étant en position *2-O-, 3-O-,* 5-*O*- et/ou 6-*O*- ; ledit substrat étant sélectionné parmi :

des aliments d'origine végétale ou animale ou une composition alimentaire comprenant desdits aliments ou un extrait desdits aliments, de préférence des extraits des céréales, des fruits, des légumes, de la viande, du poisson, des abats ;
des éléments sélectionnés parmi des métaux, des plastiques, du verre, du béton, de la pierre ;

des ustensiles, de préférence des outils ou des appareils utilisés dans l'industrie alimentaire ou hospitalière, de manière encore plus préférentielle ,des outils de chirurgie ou des prothèses ; ou
des barres de maintien ou les sièges des transports en commun.

## Patentansprüche

1. Bakterizide oder bakteriostatische Zusammensetzung für ihre Verwendung zur Behandlung oder Vorbeugung von bakteriellen Infektionen durch grampositive Bakterien, **dadurch gekennzeichnet, dass** sie ein Gemisch aus Regioisomeren und/oder Diastereoisomeren eines Alkylacetals oder eines Alkylethers von Sorbitan oder ein pharmazeutisch akzeptables Salz umfasst, wobei die Alkylgruppe zwischen 11 bis 18 Kohlenstoffatome umfasst, wobei das Alkylacetal- oder Alkyletherradikal an Position 2-*O*-, 3-*O*-, 5-*O*- und/oder 6-*O*-ist.

2. Zusammensetzung für ihre Verwendung nach Anspruch **1**, **dadurch gekennzeichnet, dass** die Alkylgruppe 11 bis 13 Kohlenstoffatome umfasst.

3. Zusammensetzung für ihre Verwendung nach dem einen oder dem anderen der Ansprüche **1** und **2**, **dadurch gekennzeichnet, dass** das Alkylacetalradikal an Position 2,3-*O*-; 3,5-*O*- oder 5,6-*O*- ist.

4. Zusammensetzung für ihre Verwendung nach einem der Ansprüche **1** bis **3**, **dadurch gekennzeichnet, dass** die grampositiven Bakterien Bakterien des Stammes der Firmicutes sind, in typischer Weise der Klasse der Bacilli, insbesondere ausgewählt aus den Bakterien der Ordnung der Lactobacillales oder der Bacillales.

5. Zusammensetzung für ihre Verwendung nach Anspruch **4**, **dadurch gekennzeichnet, dass** die grampositiven Bakterien Bakterien der Ordnung der Bacillales sind, ausgewählt aus der Familie der Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea und Turicibacteraceae.

6. Zusammensetzung für ihre Verwendung nach dem einen oder dem anderen der Ansprüche **4** und **5**, **dadurch gekennzeichnet, dass** die grampositiven Bakterien Bakterien der Familie der Listeriaceae sind wie eine Bakterie der Art der *Brochothrix* oder der *Listeria,* in typischer Weise ausgewählt aus *L. fleischmannii, L. grayi, L. innocua, L. ivanovii, L. marthii, L. monocytogenes, L. rocourtiae, L. seeligeri, L. weihenstephanensis* und *L. welshimeri.*

7. Zusammensetzung für ihre Verwendung nach dem einen oder dem anderen der Ansprüche **4** und **5**, **dadurch gekennzeichnet, dass** die grampositiven Bakterien Bakterien der Familie der Staphylococcaceae sind, ausgewählt aus den Bakterien der Art der *Staphylococcus, Gemella, Jeotgalicoccus, Macrococcus, Salinicoccus* und *Nosocomiicoccus.*

8. Zusammensetzung für ihre Verwendung nach Anspruch **7**, **dadurch gekennzeichnet, dass** die grampositiven Bakterien Bakterien der Art der *Staphylococcus* sind, ausgewählt aus *S. arlettae, S. agnetis, S. aureus, S. auricularis, S. capitis, S. caprae, S. carnosus, S. caseolyticus, S. chromogenes, S. cohnii, S. condi menti, S. delphini, S. devriesei, S. epider midis, S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis, S. pasteuri, S. pettenkoferi, S. piscifermentans, S. pseudintermedius, S. pseudolugdunensis, S. pulvereri, S. rostri, S. saccharolyticus, S. saprophyticus, S. schleiferi, S. sciuri, S. simiae, S. simulans, S. stepanovicii, S. succinus, S. vitulinus, S. warneri* und *S. xylosus.*

9. Zusammensetzung für ihre Verwendung nach Anspruch **4**, **dadurch gekennzeichnet, dass** die grampositiven Bakterien Lactobacillales sind, ausgewählt aus einer Familie der Aerococcaceae, Camobacteriaceae, Enterococcaceae, Lactobacillaceae, Leuconostocaceae und Streptococcaceae.

10. Zusammensetzung für ihre Verwendung nach Anspruch **9**, **dadurch gekennzeichnet, dass** die grampositiven Bakterien Bakterien aus der Familie der Enterococcaceae sind, ausgewählt aus den Bakterien der Art der *Bavariicoccus, Catellicoccus, Enterococcus, Melissococcus, Pilibacter, Tetragenococcus, Vagococcus.*

11. Zusammensetzung für ihre Verwendung nach Anspruch **10, dadurch gekennzeichnet, dass** die grampositiven Bakterien Bakterien der Art der *Enterococcus* sind, ausgewählt aus *E. malodoratus, E. avium, E. dur ans, E. faecalis, E. faecium, E. gallinarum, E. hirae, E. solitarius,* vorzugsweise, E. *avium, E. durans, E. faecalis* und *E. faecium.*

**12.** Zusammensetzung für ihre Verwendung nach einem der Ansprüche **1** bis **11,** wobei die Zusammensetzung in Form einer pharmazeutischen, tierarzneilichen, Hygieneprodukt- oder dermatologischen Zusammensetzung zur äußeren Anwendung vorliegt.

**13.** Zusammensetzung für ihre Verwendung nach einem der Ansprüche **1** bis **12,** wobei die Infektion durch grampositive Bakterien eine Infektion der Haut oder der Schleimhäute ist, vorzugsweise eine Infektion, ausgewählt aus einer Follikulitis, einem Abszess, einem Panaritium, einem Furunkel, einem Impetigo, einer interdigitalen Infektion, einem Anthrax (Staphylokokkenanthrax), einer Cellulitis, einer Wund-Superinfektion, einer Otitis Sinusitis, einer Hidradenitis, einer infektiösen Mastitis, einer posttraumatischen Hautinfektion und einer Infektion der verbrannten Haut.

**14.** Methode zur Desinfektion oder Vorbeugung vor der Besiedlung mit Bakterien durch grampositive Bakterien eines Substrats, die das Inkontaktversetzen des Substrats mit einer Zusammensetzung umfasst, wobei die Zusammensetzung ein Gemisch aus Regioisomeren und/oder Diastereoisomeren eines Alkylacetals oder eines Alkylethers von Sorbitan oder ein pharmazeutisch akzeptables Salz umfasst, wobei die Alkylgruppe zwischen 11 bis 18 Kohlenstoffatome umfasst, wobei das Alkylacetal- oder Alkyletherradikal an Position 2-*O*-, 3-*O*-, 5-*O*- und/oder 6-*O*- ist; wobei das Substrat ausgewählt ist aus:

Lebensmitteln pflanzlichen oder tierischen Ursprungs oder einer Lebensmittelzusammensetzung, die diese Lebensmittel oder einen Extrakt dieser Lebensmittel umfasst, vorzugsweise Extrakte von Getreide, Früchten, Gemüse, Fleisch, Fisch, Innereien; Elementen, ausgewählt aus Metallen, Kunststoffen, Glas, Beton, Stein; Utensilien, vorzugsweise Werkzeugen oder Apparaturen, die in der Lebensmittelindustrie oder im Krankenhaus verwendet werden, noch vorzugsweiser chirurgischen Werkzeugen oder Prothesen; oder
Haltegriffen oder Sitzen in öffentlichen Verkehrsmitteln.

## Claims

**1.** A bactericidal or bacteriostatic composition for use in the treatment or the prevention of bacterial infections by Gram-positive bacteria, **characterized in that** it comprises a mixture of regioisomers of an alkyl acetal or an alkyl ether of sorbitan, or a pharmaceutically acceptable salt thereof, said alkyl group comprising between 11 to 18 carbon atoms, said alkyl acetal or an alkyl ether radical being on *2-O-; 3-O- ,5-O-* and/or *6-O-* position.

**2.** The composition for use according to claim 1, **characterized in that** the alkyl group comprises 11 to 13 carbon atoms.

**3.** The composition for use according to either one of claims **1** or **2**, **characterized in that** said alkyl acetal group is in the *2,3-O-; 3, 5-O-* or *5,6-O-* position.

**4.** The composition for use according to any one of claims 1 to 3, **characterized in that** the Gram-positive bacteria are bacteria from the phylum of Firmicutes, typically of the class of Bacilli in particular chosen from bacteria of the order of Lactobacillales or Bacillales.

**5.** The composition for use according to claim **4**, **characterized in that** the Gram-positive bacteria are bacteria from the order of Bacillales chosen from the family of Alicyclobacillaceae, Bacillaceae, Caryophanaceae, Listeriaceae, Paenibacillaceae, Pasteuriaceae, Planococcaceae, Sporolactobacillaceae, Staphylococcaceae, Thermoactinomycetacea and Turicibacteriaceae.

**6.** The composition for use according to either one of claims **4** or **5**, **characterized in that** the Gram-positive bacteria are bacteria from the family of Listeriaceae such as a bacterium of the genus *Brochothrix* or *Listeria* typically chosen from *L.fleischmannii, L. grayi, L. innocua, L. ivanovii, L. marihii, L. monocytogenes, L. rocourtiae, L. seeligeri, L. weihenslephanensis* and *L. welshimeri.*

**7.** The composition for use according to either one of claims **4** or **5**, **characterized in that** the Gram-positive bacteria are bacteria from the Staphylococcaceae family chosen from bacteria from the genus *Staphylococcus, Gemella, Jeolgalicoccus, Macrococcus, Salinicoccus* and *Nosocomiicoccus.*

**8.** The composition for use according to claim **7**, **characterized in that** the Gram-positive bacteria are bacteria from the genus *Staphylococcus* chosen from *S. arlettae, S. agnelis, S. aureus, S. auricularis, S. capitis, S. caprae, S. carnosus, S. caseolyticus, S. chromogenes, S. cohnii, S. condimenti, S. delphini, S. devriesei, S. epidermidis, S.*

*equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. massiliensis, S. microti, S. muscae, S. nepalensis,S. pasteuri, S. pettenkoferi, S. piscifermentans, S. pseudintermedius, S. pseudolugdunensis,S. pulvereri, S. rostri, S. saccharolyticus, S. saprophyticus, S. schleiferi, S. sciuri, S. simiae, S. simulans, S. stepanovicii, S. succinus, S. vitulinus, S. warneri* and *S. xylosus.*

**9.** The composition for use according to claim **4**, **characterized in that** the Gram-positive bacteria are *Lactobacillales* chosen from a family of Aerococcaceae, Carnobacteriaceae, Enterococcaceae, Lactobacillaceae, Leuconostocaceae and Streptococcaceae.

**10.** The composition for use according to claim **9**, **characterized in that** the Gram-positive bacteria are bacteria from the Enterococcaceae family chosen from bacteria from the genus *Bavariicoccus, Catellicoccus, Enterococcus, Melissococcus, Pilibacler, Tetragenococcus, Vagococcus.*

**11.** The composition for use according to claim **10**, **characterized in that** the Gram-positive bacteria are bacteria from the genus *Enterococcus* chosen from *E. malodoratus, E. avium, E. durans, E. faecalis, E. faecium, E. gallinarum, E. hirae, E. solitarius,* preferably *E. avium, E. durans, E. faecalis* and *E. faecium.*

**12.** The composition for use according to any one of claims **1** to **11**, said composition being in the form of a pharmaceutical, veterinary, hygiene product or dermatological product for external use composition.

**13.** The composition for use according to any one of claims **1** to **12**, wherein the infection by Gram-positive bacteria is an infection of the skin or the mucous membranes, preferentially an infection chosen from folliculitis, an abscess, paronychia, a boil, impetigo, an infection between the digits, anthrax (staphylococcal anthrax), cellulitis, a secondary wound infection, otitis, sinusitis, hidradenitis, infectious mastitis, a post-traumatic skin infection or an infection on burnt skin.

**14.** A method for disinfection or prevention of bacterial colonization by Gram-positive bacteria of a substrate, comprising putting the substrate into contact with a composition comprising a mixture of regioisomers of an alkyl acetal or an alkyl ether of sorbitan, or a pharmaceutically acceptable salt thereof, said alkyl group comprising between 11 to 18 carbon atoms, said alkyl acetal or an alkyl ether radical being on *2-O-; 3-O- ,5-O-* and/or 6-*O*-position;
said substrate being selected from:

food of plant or animal origin, food compositions comprising thereof or an extract comprising thereof, preferably cereal, fruit, vegetable, meat, fish, offal extracts; elements selected from metals, plastics, glass, concrete or stone;
utensils, preferably tools or devices used in the food industry or hospital environment, even more preferably surgical tools and prostheses; or hand rails and seats of public transport.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014025413 A
- WO 1301375 A
- WO 1401346 A


### Littérature non-brevet citée dans la description


- **J. S. BRADLEY et al.** *Lancet Infect. Dis.,* 2007, vol. 7, 68-78
- **ANAS A. et al.** *Food Microbiology,* Avril 2015, vol. 46, 154-160
- **E. FREESE ; C. W. SHEU ; E. GALLIERS.** *Nature,* 1973, vol. 241, 321-325
- **E. G. A. VERHAEGH ; D. L. MARSHALL ; D.-H. OH.** *Int. J. Food Microbiol.,* 1996, vol. 29, 403-410
- **M. FERRER ; J. SOLIVERI ; F.J. PLOU ; N. LÓPEZ-CORTÉS ; D. REYES-DUARTE ; M. CHRISTENSEN ; J.L. COPA-PATIÑO ; A. BALLESTEROS.** *Enz. Microb. Tech.,* 2005, vol. 36, 391-398
- **J.D. MONK ; L.R. BEUCHAT ; A.K. HATHCOX.** *J. Appl. Microbiol.,* 1996, vol. 81, 7-18
- Clinical Laboratory Standards Institute. Clinical-Laboratory-Standards-Institute, 2007